# EUROPEAN PATENT SPECIFICATION

(11) **EP 2 980 038 B1**
(45) Date of publication and mention of the grant of the patent: **01.01.2020**
(21) Application number: 14775232.3
(22) Date of filing: 19.03.2014
(51) Int. Cl.: C04B 24/02, C04B 7/52, C04B 24/12, C04B 24/16, C04B 28/02, C04B 103/52, C07C 41/50, C07C 43/317

(54) **METHOD FOR IMPROVING STRENGTH OF HYDRAULIC COMPOSITION HARDENED PRODUCT**
VERFAHREN ZUR VERBESSERUNG DER FESTIGKEIT EINES GEHÄRTETEN PRODUKTES AUS EINER HYDRAULISCHEN ZUSAMMENSETZUNG
PROCÉDÉ POUR AMÉLIORER LA RÉSISTANCE DE PRODUIT DURCI DE COMPOSITION HYDRAULIQUE

(30) Priority: 26.03.2013 JP 2013064187
(43) Date of publication of application: 03.02.2016
(73) Proprietor: Kao Corporation, Chuo-ku Tokyo 103-8210 (JP)
(72) Inventor: SAGAWA, Keiichiro, Wakayama-shi, Wakayama 640-8580 (JP); SHIMODA, Masaaki, Wakayama-shi, Wakayama 640-8580 (JP); NAGASAWA, Koji, Wakayama-shi, Wakayama 640-8580 (JP)
(74) Representative: Hoffmann Eitle
(86) International application number: PCT/JP2014/057454
(87) International publication number: WO 2014/156858

(56) References cited:
- WO-A1-98/45219
- CN-A- 102 976 650
- FR-A1- 2 909 997
- JP-A- H0 517 187
- JP-A- S61 117 142
- JP-A- 2001 518 871
- JP-A- 2002 234 886
- JP-A- 2012 500 287
- US-A- 3 216 966

## Description

### Field of the invention

The present invention relates to a method for producing a hydraulic powder, an additive composition for pulverization of a hydraulic compound, and use of a strength improver composition for improving strength of a cured product of a hydraulic composition.

### Background of the invention

The early strength of concrete is important for determining the properties of concrete in early stages, such as form slip speed, frost damage resistance, and the time for removing a sheathing board, in a slip form construction method. For example, the time for a formwork to remain in place is specified in JASS5 and the Notification of the Ministry of Construction, JAPAN, No. 110, in which the minimum time for a formwork to remain in place is two to three days (for foundations, columns, walls, and the like) at an air temperature of 15°C or more. This is because the development of long-term strength is remarkably poor due to the drying of concrete after the formwork is removed, and it is said that the evaporation of water within three days is particularly significant. In order to suppress the evaporation, it is effective to accelerate the hydration reaction of cement and convert cement into a hydrate of cement from which water is not easily dried (evaporated), and it is important to highly develop 3-day strength from the viewpoint of suppressing reduction in the long-term strength due to the drying of a concrete cured product.

On the other hand, the cement industry has positively utilized the wastes (such as general refuse) and by-products produced in other industries and the like as a part of raw materials, energy sources, and products, which may vary the cement mineral composition to significantly vary the strength of cement. With respect to the quality standard of cement in Europe and China, the strength class (28-day strength in three ranks and early strength in two ranks) is distinguished from the viewpoint of strength, and the quality of cement is specified by combining these ranks. In particular, the 3-day strength representing the early strength depends on the early hydration reaction of cement and is easily influenced by the variation in the mineral composition depending on waste and the like. Therefore, it is important to develop high early strength from the viewpoint of stable production of cement. Further, blast furnace slag, fly ash, and the like which are by-products of other industries are used as an admixture (extender) of a cement product and can develop high early strength. This allows increase in the amount of the admixture, that is, reduction in the amount of clinker within the range of quality specification and is important from the viewpoint of reducing the discharge of greenhouse gas produced during the production of clinker.

In JP-A No. 2008-519752, a method of using non-treated glycerin as a cement additive for improving compressive strength of cement is disclosed.

JP-A No. 2008-542182 discloses, with regard to production of powder such as cement, a method of enhancing efficiency for pulverizing particles, which includes incorporation of a pulverization aid composition containing at least one biomass-derived polyol selected from diol, triol, and a mixture thereof to particles.

Meanwhile, with regard to a compound obtained by reacting glycerin and formaldehyde, in JP-A No. 2012-500287, as a technology of a related art, a process for producing glycerol formal by using glycerin (90%) of industrial grade and 37% formaldehyde with benzene is disclosed (Paragraph [0010] of JP-A No. 2012-500287).

Meanwhile, in JP-A No. 2008-519752, cement with compressive strength which is added with non-treated glycerin is disclosed. Furthermore, in JP-A No. 2008-542182, a cement pulverization aid containing biomass-derived polyol is disclosed.

Furthermore, in WO-A No. 2013/045419, which is published on April 4, 2013 and corresponds to EP-A No. 2574636 published on April 3, 2013, a powder composition containing glycerol formal, a copolymer, and an inorganic binder is disclosed. Furthermore, JP-A No. 2001-518871 disclosed an anti-shrinkage agent for cement composition containing at least one acetal of trihydric alcohol or polyhydric alcohol, including at least one 1,3-dioxa group.

Furthermore, CN 102 976 650 A describes a set-retarding grinding aid for set-retarding cement that is prepared by reacting a mixed solution comprising lignosulfonate, oxidant, graft modifier, and initiator, with an amination modifier and formaldehyde, followed by reaction with a polar compound such as glycerin.

### Summary of the invention

The present invention relates to a method for producing a hydraulic powder, comprising adding, during pulverization of a hydraulic compound, a strength improver composition for a hydraulic powder which contains a reaction product [hereinbelow, referred to as the component (A)] obtained by reacting (a1) glycerin [hereinbelow, referred to as the component (a1)] and (a2) formaldehyde [hereinbelow, referred to as the component (a2)], at, in terms of solid matter, 0.0005 part by mass or more and 1.0 part by mass or less relative to 100 parts by mass of the hydraulic compound.

The present invention further relates to an additive composition for pulverizing a hydraulic compound, comprising:
a strength improver composition for a hydraulic powder which contains a reaction product [hereinbelow, referred to as the component (A)] obtained by reacting (a1) glycerin [hereinbelow, referred to as the component (a1)] and (a2) formaldehyde [hereinbelow, referred to as the component (a2)]; and
at least one compound selected from the group consisting of an ethylene oxide adduct of glycerin, diethylene glycol, and triethanolamine.

The present invention further relates to the use of a strength improver composition for a hydraulic powder as a pulverization aid during pulverization of a hydraulic compound, the strength improver composition comprising a reaction product [hereinbelow, referred to as the component (A)] obtained by reacting (a1) glycerin [hereinbelow, referred to as the component (a1)] and (a2) formaldehyde [hereinbelow, referred to as the component (a2)], wherein the strength improver composition is added at, in terms of solid matter, 0.0005 part by mass or more and 1.0 part by mass or less relative to 100 parts by mass of the hydraulic compound.

### Detailed description of the invention

The present invention provides an additive composition for pulverizing a hydraulic compound which enables to obtain a cured product with high early strength. The present invention also provides a method for producing hydraulic powder which enables to obtain a cured product with high early strength, and a use of the strength improver composition for a hydraulic powder as a pulverization aid for improving strength of a cured product of a hydraulic composition.

Meanwhile, as described herein, components other than water in a subject (composition or mixed product) are defined as a "solid matter", unless specifically described otherwise. Thus, a liquid component may be contained in the solid matter.

Disclosed herein is a strength improver composition for a hydraulic powder which enables to obtain a cured product with high early strength is provided. The strength improver composition for a hydraulic powder of the present disclosure can be also added, in addition to the case of preparing a hydraulic composition, for the case of producing a hydraulic powder by pulverization of a hydraulic compound.

The reason for having a cured product with high early strength by the strength improver composition for a hydraulic powder of the present disclosure is considered to be described below, although it remains not entirely clear. When the strength improver composition for a hydraulic powder of the present disclosure is contained in a hydraulic composition having cement and water, growth of fine crystals was observed. As the mechanism for having improved early strength is speculated based on that, it is considered that, a compound containing two or more oxygen atoms with at least one ether group is obtained by reacting between polyhydric alcohol with a valency of 2 or more and an aldehyde compound or a ketone compound. Further, as the above mentioned compound adsorbs onto a surface of a hydraulic powder of a hydraulic compound via the oxygen atom, thereby rapid growth of a hydration product of C₃S being one component of the minerals of a hydraulic powder, for example, calcium hydroxide, is inhibited. Meanwhile, a compound to be crystal seed, such as calcium, is eluted from the hydraulic powder. As a result, a great number of fine crystals are generated and gaps are formed between crystals according to growth of each crystal. As a result, incorporation of water to a surface of a hydraulic powder is maintained so that hydration of a hydraulic powder proceeds at a certain rate. Thus, it is speculated that 3-day compressive strength from contact with water is improved. Meanwhile, for a reaction product with polyhydric alcohol with a valency of more than 5, adsorption onto a surface of a hydraulic powder is stronger, and as a result, it is considered that the hydration reaction of a hydraulic powder is inhibited more and thus improvement of compressive strength is deteriorated.

In the present invention, the component (A) is a reaction product which is obtained by reacting (a1) glycerol [hereinbelow, referred to as the component (a1)] and (a2) formaldehyde [hereinbelow, referred to as the component (a2)].

In the present invention, the combination of glycerin as component (a) and formaldehyde as component (b) is advantageous from the viewpoint of improving the 3-day strength of a hydraulic composition.

As for a reaction system for obtaining the component (A), a reaction system in which moisture is extracted to the outside of the system and a reaction system in which reflux of moisture is conducted can be exemplified.

With regard to the reaction system in which moisture is extracted to the outside of the system, from the viewpoint of the reactivity and improving the 3-day strength of a hydraulic composition, the component (A) is preferably a reaction product which is obtained by reacting the component (a1) and the component (a2), in terms of molar ratio of (a1)/(a2), at preferably 0.9/1.0 or more, and also preferably 1.1/1.0 or less and more preferably 1.0/1.0 or less.

With regard to the reaction system in which moisture is extracted to the outside of the system for obtaining the component (A), the reaction is conducted at a reaction temperature of preferably 80°C or higher, more preferably 90°C or higher, and even more preferably 100°C from the viewpoint of the reactivity and improving the 3-day strength of a hydraulic composition. The reaction can be conducted at atmospheric pressure.

With regard to the reaction system in which moisture is extracted to the outside of the system for obtaining the component (A), the reaction is conducted for the reaction time of preferably 1 hour or more, more preferably 2 hours or more, and even more preferably 4 hour or more, and also preferably 24 hours or less, more preferably 10 hours or less, and even more preferably 6 hours or less from the viewpoint of the reactivity and improving the 3-day strength of a hydraulic composition.

With regard to the reaction system in which reflux of moisture is conducted, from the viewpoint of the reactivity and improving the 3-day strength of a hydraulic composition, the component (A) is preferably a reaction product which is obtained by reacting the component (a1) and the component (a2), in terms of molar ratio of (a1)/(a2), at preferably 1.0/1.0 or more, and also preferably 4.0/1.0 or less and more preferably 2.0/1.0 or less.

With regard to the reaction system in which reflux of moisture is performed for obtaining the component (A), the reaction is performed at a reaction temperature of preferably 80°C or lower, more preferably 50°C or lower, and even more preferably 20°C or lower from the viewpoint of the reactivity and improving the 3-day strength of a hydraulic composition. The reaction can be performed at atmospheric pressure.

With regard to the reaction system in which reflux of moisture is performed for obtaining the component (A), the reaction is performed for the reaction time of preferably 1 hour or more, more preferably 2 hours or more, and even more preferably 4 hours or more, and also preferably 24 hours or less, more preferably 10 hours or less, and even more preferably 6 hours or less from the viewpoint of the reactivity and improving the 3-day strength of a hydraulic composition.

Furthermore, the component (A) is, from the viewpoint of the reactivity and improving the 3-day strength of a hydraulic composition, preferably a reaction product which is obtained by reacting the component (a1) and the component (a2) in the presence of a catalyst. Examples of the catalyst include an acid catalyst and an alkali catalyst. Examples of the acid catalyst include inorganic acid, organic acid, and solid acid. Examples of the inorganic acid include hydrochloric acid, nitric acid, phosphoric acid, sulfuric acid, boric acid, and hydrogen fluoride acid. Examples of the organic acid include formic acid, acetic acid, citric acid, oxalic acid, paratoluene sulfonic acid, trifluoromethane sulfonic acid, and fluorosulfonic acid. Examples of the alkali catalyst include alkali metal hydroxide such as lithium hydroxide, sodium hydroxide, potassium hydroxide, rubidium hydroxide, or cesium hydroxide, alkali metal carbonate such as lithium carbonate, sodium carbonate, potassium carbonate, rubidium carbonate, or cesium carbonate, and alkali metal hydrogen carbonate such as lithium hydrogen carbonate, sodium hydrogen carbonate, potassium hydrogen carbonate, rubidium hydrogen carbonate, or cesium hydrogen carbonate. The catalyst is used, in terms of molar ratio of the catalyst/the component (a2), at preferably 0.001/1 or more, more preferably 0.002/1 or more, and even more preferably 0.005/1 or more, and also preferably 0.1/1 or less, more preferably 0.05/1 or less, and even more preferably 0.01/1 or less relative to the component (a2).

In order to obtain the reaction product of the component (A), it is preferable to have the following steps:
(1) a step of mixing the component (a1), the component (a2), and a reaction catalyst to form a mixed product;
(2) a step of adjusting the mixed product obtained from (1) to a reaction temperature and maintaining the temperature for 1 hour or more;
(3) a step of cooling the mixed product after the step of (2); and
(4) a step of neutralizing the mixed product either after or during the step (3).

The component (A) is generally obtained as a liquid reaction product. In the present invention, the component (A) can be directly used as a strength improver for a hydraulic powder. The strength improver for a hydraulic powder containing the component (A) may be admixed with another component, for example, water, the component (B), or the component (C) described below so as to be used as a strength improver composition for a hydraulic powder. Specific examples thereof include a strength improver composition for a hydraulic powder containing the component (A) and the component (B) and a strength improver composition for a hydraulic powder containing the component (A), the component (B), and the component (C).

In the component (A), the content of the solid matter is preferably 20% by mass or more, more preferably 30% by mass or more, and even more preferably 40% by mass or more, and also preferably 100% by mass or less. The solid matter content in the reaction product varies depending on the reaction conditions for obtaining the component (A) (a reaction system in which moisture is extracted to the outside of the system and a reaction system in which reflux of moisture is performed). In the case of conducting a reaction according to a reaction system in which moisture is extracted to the outside of the system, as the component (A), a reaction product having a solid matter content of preferably 80% by mass or more, more preferably 90% by mass or more, and even more preferably 95% by mass or more can be obtained. Furthermore, in the case of conducting a reaction according to a reaction system in which reflux of moisture is performed, as the (A), a reaction product having a solid matter content of preferably 90% by mass or less and more preferably 80% by mass or less can be obtained.

In the reaction product, an unreacted component is contained as a component other than water. In the reaction product of the component (A), content of the unreacted component (a1) according to quantitative gas chromatography analysis is, for a reaction system in which moisture is extracted to the outside of the system, preferably 20% by mass or less, more preferably 15% by mass or less, and even more preferably 10% by mass or less, and also 0% by mass or more in terms of solid matter, in the solid matter of the reaction product from the viewpoint of improving the 3-day strength of a hydraulic composition.

For a reaction system in which reflux of moisture is conducted, the content of the unreacted component (a1) in the reaction product of the component (A) is preferably 60% by mass or less, more preferably 50% by mass or less, and even more preferably 40% by mass or less, and also 0% by mass or more in terms of solid matter, in the solid matter of the reaction product from the viewpoint of improving the 3-day strength of a hydraulic composition. Furthermore, from the viewpoint or easy reaction between the component (a1) and the component (a2), it is preferably 10% by mass or more, more preferably 20% by mass or more, and even more preferably 30% by mass or more.

In any of those reaction systems, the content of the unreacted component (a1) is measured by quantitative gas chromatography analysis under the following conditions. Meanwhile, the sampled reaction solution is precisely weighed with liquid squalane as an internal reference, derivatized with an excess amount of trimethyl silylating agent, and then subjected to the measurement.

### [Conditions for gas chromatography measurement]

Apparatus: HP6850 manufactured by Agilent Technologies, Inc.
Column: Frontier Ultra-Alloy-1 (30 m × 0.25 mm id × 0.15 µm film thickness)
Apparatus conditions:
- Injection temp.: 300°C
- Detector temp.: 350°C
- H₂ flow: 30 mL/min
- Air flow: 300 mL/min
- He flow: 28 mL/min
- Injection volume: 2.0 µL
- Split mode: Split ratio = 50/1
- Total Flow rate: 104 mL/min (He)
Temperature program
- Initial temp.: 40°C
- Initial time: 5 min
- Increasing rate: 10°C/min
- Final temp.: 350°C

In the strength improver composition for a hydraulic powder of the present disclosure, the content of the solid matter containing the component (A), the component (B) and the component (C) that are described below is, from the viewpoint of storing and transporting the strength improver composition for a hydraulic powder, preferably 8% by mass or more, more preferably 18% by mass or more, and even more preferably 20% by mass or more. From the viewpoint of workability of addition operation of the strength improver composition for a hydraulic powder or the like, the content is preferably 100% by mass or less, more preferably 90% by mass or less, and even more preferably 80% by mass or less.

Furthermore, the ratio of the component (A) to the solid matter of the strength improver composition for a hydraulic powder is, from the viewpoint of improving the 3-day strength of a hydraulic composition, preferably 3% by mass or more and more preferably 8% by mass or more, and also from the viewpoint of improving the 3-day strength of a hydraulic composition, preferably 100% by mass or less, more preferably 80% by mass or less, and even more preferably 75% by mass or less.

When the ratio of the component (A) to the solid matter of the strength improver composition for a hydraulic powder is preferably 55% by mass or more and more preferably 80% by mass or more, the content of the solid matter in the strength improver composition for a hydraulic powder is preferably 20% by mass or more, more preferably 30% by mass or more, and even more preferably 40% by mass or more. From the viewpoint of workability of addition operation of the strength improver composition for a hydraulic powder or the like, the content is preferably 100% by mass or less, more preferably 80% by mass or less, and even more preferably 60% by mass or less.

The content of the component (A) in the strength improver composition for a hydraulic powder for a case in which only the component (A) is used is, from the viewpoint of storing and transporting the strength improver composition for a hydraulic powder, preferably 20% by mass or more, more preferably 30% by mass or more, and even more preferably 40% by mass or more. Furthermore, from the viewpoint of workability of addition operation of the strength improver composition for a hydraulic powder or the like, the content is preferably 100% by mass or less, more preferably 80% by mass or less, and even more preferably 60% by mass or less.

For a case in which only the component (A) is used, the strength improver composition for a hydraulic powder may contain water from the viewpoint of workability of addition operation or the like. Content of water is, from the viewpoint of workability of addition operation or the like, preferably 20% by mass or more and more preferably 40% by mass or more in the strength improver composition for a hydraulic powder. From the viewpoint of storing and transporting the strength improver composition for a hydraulic powder, the content of water is preferably 80% by mass or less, more preferably 70% by mass or less, and even more preferably 60% by mass or less.

The strength improver composition for a hydraulic powder of the present disclosure may contain, as the component (B), at least one compound selected from urea-formaldehyde condensate and hydroxymethane sulfonate from the viewpoint of improving the 3-day strength of a hydraulic composition. Examples of the urea-formaldehyde condensate include tetrahydro-4H-1,3,5-oxadiazin-4-one. Furthermore, the hydroxymethane sulfonate is preferably an alkali metal salt such as sodium salt and potassium salt. It is possible that the component (A) and the component (B) are admixed with each other in advance to produce a strength improver composition for a hydraulic powder and can be used after being added to a hydraulic powder. It is also possible that the component (A) and the component (B) are separately added to a hydraulic powder and used.

In case of that the component (B) is contained, the content of the component (A) in the solid matter of the strength improver composition for a hydraulic powder is, from the viewpoint of improving the 3-day strength of a hydraulic composition, preferably 10% by mass or more, more preferably 25% by mass or more, and even more preferably 35% by mass or more, and also preferably 90% by mass or less, more preferably 70% by mass or less, and even more preferably 60% by mass or less. Furthermore, for a case in which the component (B) is contained, the ratio of the component (B) to the solid matter of the strength improver composition for a hydraulic powder is, from the viewpoint of improving the 3-day strength of a hydraulic composition, preferably 10% by mass or more, more preferably 30% by mass or more, and even more preferably 40% by mass or more, and also preferably 90% by mass or less, more preferably 75% by mass or less, and even more preferably 65% by mass or less.

In case of that the component (B) is contained, the mass ratio of the component (B) to the component (A) (when it is converted in terms of solid matter) is, from the viewpoint of improving the 3-day strength of a hydraulic composition, preferably more than 0/100 in terms of (B)/(A), that is, the component (B) is present at more than 0 part by mass, preferably 20/80 or more, more preferably 45/55 or more, and even more preferably 50/50 or more and also preferably 80/20 or less, more preferably 70/30 or less, and even more preferably 60/40 or less relative to 100 parts by mass of the component (A).

The content of the component (A) for the strength improver composition for a hydraulic powder containing both the component (A) and the component (B) is, from the viewpoint of improving the 3-day strength of a hydraulic composition, preferably 3% by mass or more, more preferably 5% by mass or more, and even more preferably 8% by mass or more, and also preferably 60% by mass or less, more preferably 50% by mass or less, even more preferably 45% by mass or less, even more preferably 30% by mass or less, and even more preferably 20% by mass or less.

The content of the component (B) for the strength improver composition for a hydraulic powder containing both the component (A) and the component (B) is, from the viewpoint of improving the 3-day strength of a hydraulic composition, preferably 5% by mass or more and more preferably 10% by mass or more, and also preferably 60% by mass or less, more preferably 50% by mass or less, even more preferably 45% by mass or less, even more preferably 30% by mass or less, and even more preferably 15% by mass or less.

The total content of the component (A) and the component (B) for the strength improver composition for a hydraulic powder containing both the component (A) and the component (B) is preferably 8% by mass or more, more preferably 15% by mass or more, even more preferably 18% by mass or more, and even more preferably 20% by mass or more from the storing and transporting the strength improver, composition for a hydraulic powder. From the viewpoint of workability of addition operation of the strength improver composition for a hydraulic powder or the like, the content of the component (A) and component (B) is preferably 100% by mass or less, more preferably 90% by mass or less, even more preferably 80% by mass or less, and even more preferably 60% by mass or less.

The strength improver composition for a hydraulic powder containing both the component (A) and the component (B) may contain water from the viewpoint of workability of addition operation or the like. The content of water in the strength improver composition is preferably 10% by mass or more, more preferably 20% by mass or more, and even more preferably 40% by mass or more, and from the viewpoint of storing and transporting the strength improver composition for a hydraulic powder, preferably 95% by mass or less, more preferably 92% by mass or less, even more preferably 82% by mass or less, and even more preferably 80% by mass or less. The form of the strength improver composition for a hydraulic powder of the present disclosure is preferably liquid form. The strength improver composition for a hydraulic powder of the present disclosure may also contain an anti-foaming agent described below.

As the strength improver composition for a hydraulic powder of the present disclosure is co-present with a hydraulic powder, it improves the strength of a cured product of the hydraulic powder. In general, a hydraulic powder is used as a hydraulic composition containing the powder and water. Examples of the hydraulic powder include cement. Examples of the cement include an ordinary Portland cement, an early strength Portland cement, a high early strength Portland cement, a sulfate-resistant Portland cement, a low heat Portland cement, a white Portland cement, and an eco cement (for example, JIS R5214). The cement may include an admixture such as blast furnace slag, fly ash, silica fume, volcano ash, and silicate white clay as other hydraulic powder. Furthermore, non-hydraulic limestone fine powder may be also included. As for the admixture, at least one selected from blast furnace slag, fly ash, and silica fume is preferable. At least one selected from blast furnace slag and fly ash is more preferable. A mixed cement admixed with cement, for example, silica fume cement or furnace cement may be used. Furthermore, the admixture may be used without being mixed with cement. The hydraulic powder in which the strength improver composition for a hydraulic powder of the present disclosure is used preferably contains an admixture as a material which has no curing property by itself but, when combined with a material having a curing property upon reaction with water or an alkali material, forms a hydrate according to an interaction via water to obtain curing. Namely, the strength improver composition for a hydraulic powder of the present disclosure is preferable for a hydraulic powder which contains at least one substance (admixture) selected from blast furnace slag, fly ash, and silica fume and also, from the viewpoint of improving the 3-day strength of a hydraulic composition, for a hydraulic powder which contains at least one substance (admixture) selected from blast furnace slag, fly ash, and silica fume at preferably 10% by mass or more and more preferably 30% by mass or more, and also preferably 80% by mass or less, more preferably 70% by mass or less, and even more preferably 50% by mass or less.

The strength improver composition for a hydraulic powder of the present disclosure is used, in terms of solid matter, at ratio of 0.0005 part by mass or more and also 1.0 part by mass or less relative to 100 parts by mass of a hydraulic powder from the viewpoint of improving the 3-day strength of a hydraulic composition and suppressing a long-term strength decrease. From the viewpoint of improving the 3-day strength of a hydraulic composition, it is used, in terms of solid matter, at ratio of more preferably 0.001 part by mass or more, even more preferably 0.003 part by mass or more, even more preferably 0.008 part by mass or more, even more preferably 0.012 part by mass or more, and even more preferably 0.020 part by mass or more relative to 100 parts by mass of a hydraulic powder. Furthermore, from the viewpoint of improving the 3-day strength of a hydraulic composition and suppressing a long-term strength decrease, it is used, in terms of solid matter, at ratio of more preferably 0.5 part by mass or less, even more preferably 0.3 part by mass or less, and even more preferably 0.1 part by mass or less relative to 100 parts by mass of a hydraulic powder.

For applying the strength improver composition for a hydraulic powder of the present disclosure to a hydraulic powder, the aforementioned composition may be added to a hydraulic compound during the step of producing a hydraulic powder. In general, the hydraulic powder is produced by pulverizing a hydraulic compound. When the strength improver composition for a hydraulic powder of the present disclosure is added during pulverization of a hydraulic compound, the strength improver composition for a hydraulic powder of the present disclosure is added, in terms of solid matter, at 0.0005 part by mass or more, and from the viewpoint of improving the 3-day strength of a hydraulic composition, preferably 0.001 part by mass or more, more preferably 0.003 part by mass or more, even more preferably 0.008 part by mass or more, even more preferably 0.012 part by mass or more, and even more preferably 0.020 part by mass or more, and also 1.0 part by mass or less, more preferably 0.5 part by mass or less, even more preferably 0.3 part by mass or less, and even more preferably 0.1 part by mass or less relative to 100 parts by mass of a hydraulic compound. For example, as a method for producing a hydraulic powder including a step of pulverizing a hydraulic compound in the presence of the strength improver composition for a hydraulic powder of the present disclosure, a method for producing a hydraulic powder in which the strength improver composition for a hydraulic powder of the present disclosure is present at ratio of 0.0005 part by mass or more, preferably 0.001 part by mass or more, more preferably 0.003 part by mass or more, even more preferably 0.008 part by mass or more, even more preferably 0.012 part by mass or more, and even more preferably 0.020 part by mass or more, and also 1.0 part by mass or less, more preferably 0.5 part by mass or less, even more preferably 0.3 part by mass or less, and even more preferably 0.1 part by mass or less relative to 100 parts by mass of a hydraulic compound is provided. In that case, from the viewpoint of the property of pulverizing a hydraulic compound, the hydraulic compound can be pulverized in the presence of at least one compound selected from an ethylene oxide adduct of glycerin, diethylene glycol, and triethanolamine [hereinbelow, referred to as the component (C)]. The component (C) is preferably an ethylene oxide adduct of glycerin. In the ethylene oxide adduct of glycerin, the average added mole number of ethylene oxide is preferably 0.5 or more and 3 or less.

The present invention provides an additive composition for pulverization of a hydraulic compound containing the strength improver composition for a hydraulic powder of the present disclosure and the component (C). When the component (C) is used in combination, the strength improver composition for a hydraulic powder preferably contains the component (A) and the component (B) in the additive composition for pulverization. Furthermore, the mass ratio of the total of the component (A) and the component (B) to the component (C) (when it is converted in terms of solid matter) in the strength improver composition for a hydraulic powder of the present disclosure is, from the viewpoint of improving the property for pulverizing a hydraulic compound and from the viewpoint of improving the 3-day strength of a hydraulic composition, preferably 10/90 or more, more preferably 20/80 or more, and even more preferably 40/60 or more and also preferably 90/10 or less, more preferably 80/20 or less, and even more preferably 60/40 or less in terms of [total of the component (A) and the component (B)]/the component (C). The additive composition for pulverization may be used as a composition containing water such as an aqueous solution. In that case, the total concentration of the strength improver composition for a hydraulic powder and the component (C) is, from the viewpoint of improving the handling property of a mixed product as a liquid mixed product having low viscosity, preferably 20% by mass or more, more preferably 30% by mass or more, and even more preferably 35% by mass or more, and also preferably 99% by mass or less and more preferably 80% by mass or less.

According to the method for improving the strength of a cured product of a hydraulic composition of the present disclosure, the strength improver composition for a hydraulic powder is added to a hydraulic composition. As for the timing for addition, it may be during the preparation of a hydraulic composition, and during or after the pulverization of a hydraulic compound during production of the hydraulic powder.

In the method for producing a hydraulic powder of the present invention, a hydraulic compound is ground to obtain the hydraulic powder. The hydraulic compound refers to a substance having a property of reacting with water to be hardened and a compound that does not have hardenability as a single substance, but forms a hydrate and is hardened by the interaction through water when two or more substances are used in combination. Generally, in the hydraulic compound, an oxide of an alkaline earth metal and an oxide such as SiO₂, Al₂O₃, Fe₂O₃, TiO₂, P₂O₅, and ZnO form a hydrate under ordinary temperature or hydrothermal conditions. The components of the hydraulic compound, for example cement, include 3CaO·SiO₂ (C₃S: alite), 2CaO·SiO₂ (C₂S: belite), 3CaO·Al₂O₃ (C₃A: calcium aluminate), and 4CaO·Al₂O₃·Fe₂O₃ (C₄AF: calcium alumino ferrite), as components. Further, examples of the admixture used with cement include one or more substances selected from blast furnace slag, fly ash, and silica fume. Examples of the hydraulic compound include minerals contained in cement (C₃S, C₂S, C₃A, and C₄AF), slag, fly ash, limestone, iron slag, gypsum, alumina, incinerated ash, unslaked lime, and slaked lime, which can be used as a raw material of a hydraulic powder. The hydraulic compound preferably contains one or more substances (admixtures) selected from the group consisting of blast furnace slag, fly ash, and silica fume. Further, the hydraulic compound contains one or more substances (admixtures) selected from the group consisting of blast furnace slag, fly ash, and silica fume in an amount of preferably 10% by mass or more, more preferably 30% by mass or more, and preferably 80% by mass or less, more preferably 70% by mass or less, even more preferably 50% by mass or less, from the viewpoint of improving the 3-day strength of the hydraulic composition.

When Portland cement is obtained as a hydraulic powder, it is produced as follows, for example: clinker as a hydraulic compound obtained by calcination of a raw material such as limestone, clay, and iron slag (also referred to as cement clinker, and it may also contain gypsum) is pre-pulverized, preferably with the aforementioned admixture, added with a suitable amount of gypsum, and subjected to finishing pulverization to produce a powder having a specific surface area, for example, a blaine value of 2500 cm²/g or more. The strength improver composition for a hydraulic powder of the present disclosure is used as an additive during pulverization of the above-mentioned hydraulic compound, preferably a clinker, and preferably as an additive for finishing pulverization. From the viewpoint of improving the 3-day strength of a hydraulic composition, the strength improver composition for a hydraulic powder is used such that it is present, in terms of solid matter of the strength improver composition for a hydraulic powder, at 0.0005 part by mass or more, more preferably 0.005 part by mass or more, and even more preferably 0.01 part by mass or more, and also 1.0 part by mass or less, more preferably 0.5 part by mass or less, even more preferably 0.1 part by mass or less, and even more preferably 0.05 part by mass or less relative to 100 parts by mass of a hydraulic compound as a raw material used for the pulverization. Furthermore, from the viewpoint of improving the property of pulverizing a hydraulic compound and from the viewpoint of improving the 3-day strength of a hydraulic composition, a mixed product of the strength improver composition for a hydraulic powder and the component (C) can be used as an additive during pulverization of the above-mentioned hydraulic compound, preferably a clinker, and preferably as an additive in finishing pulverization. Furthermore, the amount is based on the solid matter amount of the strength improver composition for a hydraulic powder that is present during the step of pulverizing a hydraulic compound, and specifically, it is based on the solid matter amount of the strength improver composition for a hydraulic powder that is present until the pulverization of a hydraulic compound is completed, or until the desired blaine value is reached.

With regard to the method for producing a hydraulic powder of the present invention, the conditions for pulverization may be adjusted depending on raw materials, use, or the like such that a powder with suitable particle diameter is obtained. It is generally preferable that the hydraulic compound, for example, clinker, be pulverized until it turns into a powder with a blaine value of preferably 2500 cm²/g or more and more preferably 3000 cm²/g or more, and also preferably 5000 cm²/g or less and more preferably 4000 cm²/g or less. The desired Blaine value can be obtained by adjusting the pulverization time, for example. There is a tendency that the Blaine value increases as the pulverization time is extended, and it decreases as the pulverization time is shortened.

In the present invention, the pulverization device used for pulverization of a hydraulic compound is not particularly limited, and examples thereof include a ball mill which is generally used for pulverization of cement. Material of a pulverization medium (pulverizing ball) of the device preferably has hardness which is the same or greater than that of a subject for pulverization (for example, calcium aluminate for cement clinker). Examples of a generally obtainable commercial product include steel, stainless steel, alumina, zirconia, titania, and tungsten carbide.

From the viewpoint of suppressing a decrease in strength caused by increased air amount in a hydraulic composition, an anti-foaming agent can be additionally used in combination. Furthermore, by having an anti-foaming agent present at the time of pulverization of a hydraulic compound, the anti-foaming agent can be evenly distributed on a surface of the hydraulic powder to be obtained so that the aforementioned suppressing effect can be more effectively exhibited. Namely, according to a method for producing a hydraulic powder including a step of pulverizing a hydraulic compound in the presence of the strength improver composition for a hydraulic powder and an anti-foaming agent, a decrease in compressive strength of a hydraulic composition, which is caused by increased air amount, can be suppressed.

Preferred examples of the anti-foaming agent include a silicone anti-foaming agent, a fatty acid ester anti-foaming agent, and an ether anti-foaming agent. As for the silicone anti-foaming agent, dimethylpolysiloxane is more preferable. As for the fatty acid ester anti-foaming agent, polyalkylene glycol fatty acid ester is more preferable. As for the ether anti-foaming agent, polyalkylene glycol ether is more preferable.

The hydraulic composition using a hydraulic powder obtained by the production method of the present invention has improved compressive strength at curing. Examples of the hydraulic powder include Portland cement, alumina cement, blast furnace slag, fly ash, limestone, and gypsum. Portland cement is preferable. A hydraulic powder containing the aforementioned admixture is preferable.

The hydraulic powder obtained by the production method of the present invention can be used as a material for a concrete structure and a concrete product. The concrete using the hydraulic powder obtained by the production method of the present invention has improved compressive strength 3 days after contact with water. For example, even if a hydraulic powder (such as blast furnace slag, fly ash, and limestone) having a low early age strength after contact with water is blended with or partially substituted for the hydraulic powder obtained by the production method of the present invention, compressive strength 3 days after contact with water that is equal to or higher than that in the case of using a hydraulic powder to which the present invention is not applied can be obtained, which is an advantage of the hydraulic powder obtained by the production method of the present invention.

According to the present disclosure, a hydraulic composition containing the strength improver composition for a hydraulic powder of the present disclosure, a hydraulic powder, an aggregate, and water, in which the content of the strength improver composition for a hydraulic powder is, from the viewpoint of improving the 3-day strength of a hydraulic composition, 0.0005 part by mass or more and 1.0 parts by mass or less in terms of solid matter relative to 100 parts by mass of a hydraulic powder is provided.

In the hydraulic composition of the present disclosure, the content of the strength improver composition for a hydraulic powder is, from the viewpoint of improving the 3-day strength of a hydraulic composition, 0.0005 part by mass or more, preferably 0.005 part by mass or more in terms of solid matter, and it is, from the viewpoint of improving the 3-day strength of a hydraulic composition, preferably 0.001 part by mass or more, more preferably 0.003 part by mass or more, even more preferably 0.008 part by mass or more, even more preferably 0.012 part by mass or more, and even more preferably 0.020 part by mass or more, and also 1.0 parts by mass or less relative to 100 parts by mass of a hydraulic powder. Furthermore, from the viewpoint of improving the 3-day strength of a hydraulic composition, it is preferably 0.5 part by mass or less, even more preferably 0.3 part by mass or less, and even more preferably 0.1 part by mass or less.

As for the hydraulic powder, those described above can be used. The hydraulic powder preferably contains at least one substance (admixture) selected from blast furnace slag, fly ash, and silica fume. It is preferably a hydraulic powder which contains at least one substance (admixture) selected from blast furnace slag, fly ash, and silica fume at preferably 10% by mass or more and more preferably 30% by mass or more, and also preferably 80% by mass or less, more preferably 70% by mass or less, and even more preferably 50% by mass or less from the viewpoint of improving the 3-day strength of a hydraulic composition. The content of a hydraulic powder in the hydraulic composition of the present disclosure is, from the viewpoint of separation resistance of a hydraulic composition and strength after hardening, preferably 300 kg/m³ or more and more preferably 350 kg/m³ or more per volume of the hydraulic composition. Furthermore, from the viewpoint of suppressing cracks that are caused by hydration heat of a hydraulic composition, it is preferably 450 kg/m³ or less and more preferably 430 kg/m³ or less.

As for the aggregate, an aggregate selected from fine aggregate and coarse aggregate can be exemplified. Examples of the fine aggregate include those defined by JIS A0203-2302. Examples of the fine aggregate include river sand, land sand, mountain sand, sea sand, limestone sand, silicate and sand obtained by crushing them, fine aggregate of blast furnace slag, fine aggregate of ferronickel slag, light weight fine aggregate (both the artificial and natural aggregate), and recycled fine aggregate. Further, examples of the coarse aggregate include those defined by JIS A0203-2303. Examples of the coarse aggregate include river gravel, land gravel, mountain gravel, sea gravel, limestone gravel, gravels obtained by crushing them, coarse aggregate of blast furnace slag, coarse aggregate of ferronickel slag, light weight coarse aggregate (both the artificial aggregate and natural aggregate), and recycled coarse aggregate. Different types of the fine aggregate and coarse aggregate can be mixed and used, or those with the same type can be used.

The content of the aggregate in the hydraulic composition of the present disclosure is, from the viewpoint of separation resistance of a hydraulic composition and workability, preferably 1700 kg/m³ or more and more preferably 1720 kg/m³ or more and also preferably 1800 kg/m³ or less and more preferably 1760 kg/m³ or less per volume of the hydraulic composition.

The volume ratio of the fine aggregate (s) to the aggregate (a) in the hydraulic composition of the present disclosure [s/a × 100 (%)] is, from the viewpoint of separation resistance and workability of a hydraulic composition, preferably 45% or more and more preferably 47% or more and also preferably 55% or less and even more preferably 53% or less.

The mass ratio of water (W) to the hydraulic powder (C) in the hydraulic composition of the present disclosure [W/C × 100 (%)] is, from the viewpoint of the fluidity of the hydraulic composition, preferably 35% or more and more preferably 38% or more. From the viewpoint of the strength of a hydraulic composition after hardening, it is preferably 55% or less and even more preferably 52% or less.

The hydraulic composition of the present disclosure can contain a dispersing agent from the viewpoint of increasing fluidity. Examples of the dispersing agent include a phosphate-based polymer, a polycarboxylic acid-based copolymer, a sulfonic acid-based copolymer, a naphthalene-based polymer, a melamine-based polymer, a phenolic polymer, and a lignin-based polymer. The dispersing agent may be a chemical admixture in which other components are blended.

As the dispersing agent, a dispersing agent selected from a polycarboxylic acid-based copolymer and a naphthalene-based polymer is preferred, and a polycarboxylic acid-based copolymer is more preferred, from the viewpoint of suppressing the delay of hardening of the hydraulic composition. Examples of the polycarboxylic acid-based copolymer which can be used include a copolymer of a monoester of a polyalkylene glycol and (meth)acrylic acid and a carboxylic acid such as (meth)acrylic acid (such as a compound described in JP-A No. 8-12397), a copolymer of an unsaturated alcohol having a polyalkylene glycol and a carboxylic acid such as (meth)acrylic acid, and a copolymer of an unsaturated alcohol having a polyalkylene glycol and a dicarboxylic acid such as maleic acid. Here, (meth)acrylic acid means a carboxylic acid selected from acrylic acid and methacrylic acid.

As the polycarboxylic acid-based copolymer, a copolymer produced from polymerization of the monomer (1) represented by the following formula (1) and the monomer (2) represented by the following formula (2) can be used [hereinbelow, it is referred to as the polycarboxylic acid copolymer (I)]. 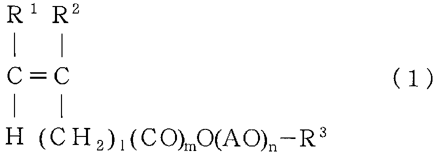 [in the formula,
R¹ and R²: a hydrogen atom or a methyl group,
l: number of 0 or more and 2 or less,
m: number of 0 or 1,
AO: an alkyleneoxy group having 2 or more and 4 or less carbon atoms,
n: an average added mole number of AO, which is a number of 5 or more and 150 or less, and
R³: a hydrogen atom or an alkyl group having 1 or more and 4 or less carbon atoms]. 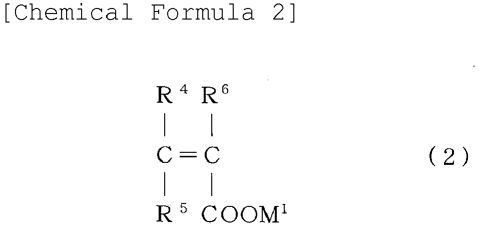
[in the formula,
R⁴, R⁵, and R⁶: a hydrogen atom, a methyl group, or (CH₂)ₘ₁COOM²,
M¹ and M²: a hydrogen atom, an alkali metal, an alkaline earth metal (1/2 atom), ammonium, alkyl ammonium, or substituted alkyl ammonium, and
m1: number of 0 or more and 2 or less.

Meanwhile, (CH₂)ₘ₁COOM² and COOM¹ may together form an anhydride].

From the viewpoint of fluidity of the hydraulic composition, in Formula (1), AO preferably represents an alkyleneoxy group (ethyleneoxy group) having 2 or 3 carbon atoms, and more preferably having 2 carbon atoms.

From the viewpoint of suppressing delayed hardening of a hydraulic composition, n preferably represents the number of not less than 9, more preferably not less than 20, even more preferably not less than 50, and even more preferably not less than 70. From the viewpoint of early fluidity of a hydraulic composition, n preferably represents the number of not more than 150, and more preferably not more than 130.

When m is 0, l is preferably 1 or 2. When m is 1, l is preferably 0. From the viewpoint of polymerizability of the copolymer during polymerization, m is preferably 1. When m is 0, R³ is preferably a hydrogen atom from the viewpoint of ease of production of the monomer. When m is 1, R³ is preferably an alkyl group having 1 or more and 4 or less carbon atoms from the viewpoint of ease of production of the monomer, and more preferably a methyl group from the viewpoint of water solubility.

Examples of the monomer (1) which can be used include an ester of a polyalkylene glycol and (meth)acrylic acid and an ether in which an alkylene oxide is added to an alkenyl alcohol. The monomer (1) is preferably an ester of a polyalkylene glycol and (meth)acrylic acid from the viewpoint of polymerizability of the copolymer during polymerization.

Examples of the ester of a polyalkylene glycol and (meth)acrylic acid which can be used include an ester of a one end-blocked alkylene glycol and (meth)acrylic acid. Specific examples thereof which can be used include one or more of methoxy polyethylene glycol acrylate, methoxy polyethylene glycol methacrylate, ethoxy polyethylene glycol acrylate, and ethoxy polyethylene glycol methacrylate.

Further, examples of the ether in which an alkylene oxide is added to an alkenyl alcohol which can be used include an ethylene oxide adduct of allyl alcohol. Specific examples thereof which can be used include an ethylene oxide adduct of methallyl alcohol and an ethylene oxide adduct of 3-methyl-3-buten-1-ol.

Examples of the monomer (2) which can be used include one or more selected from acrylic acid and a salt thereof, methacrylic acid and a salt thereof, maleic acid and a salt thereof, and maleic anhydride. When m of the monomer (1) is 1, the monomer (2) is preferably methacrylic acid or a salt thereof from the viewpoint of polymerizability of the copolymer during polymerization; and when m of the monomer (1) is 0, the monomer (2) is preferably maleic acid or a salt thereof and maleic anhydride from the viewpoint of polymerizability of the copolymer during polymerization.

In the polycarboxylic acid-based copolymer (I), the molar ratio of the monomer (1) to the monomer (2), monomer (1)/monomer (2), is preferably 3/97 or more, more preferably 5/95 or more, and even more preferably 10/90 or more, and also preferably 70/30 or less, more preferably 50/50 or less, and even more preferably 30/70 or less from the viewpoint of improving the early fluidity of a hydraulic composition.

Furthermore, in the entire monomers contained in the polycarboxylic acid-based copolymer (I), the total ratio of the monomer (1) and the monomer (2) is preferably 50% by mol or more and more preferably 80% by mol or more, and also preferably 100% by mol or less and more preferably 100% by mol from the viewpoint of improving the early fluidity of a hydraulic composition. Meanwhile, as a constituent monomer other than the monomer (1) and the monomer (2), at least one selected from alkyl ester of unsaturated carboxylic acid or the like can be used.

The weight average molecular weight of the polycarboxylic acid copolymer (I) is preferably 10000 or more, more preferably 35000 or more, and even more preferably 50000 or more from the viewpoint of improving the early fluidity of a hydraulic composition. Furthermore, the weight average molecular weight of the polycarboxylic acid copolymer (I) is, from the viewpoint of lowering the viscosity of a hydraulic composition, preferably 100000 or less, more preferably 80000 or less, and even more preferably 70000 or less. The weight average molecular weight is measured by gel permeation chromatography (GPC) method under the following conditions.

### [GPC conditions]

Apparatus: High speed GPC apparatus HLC-8320GPC (manufactured by TOSOH CORPORATION)
Column: G4000PWXL + G2500PWXL (manufactured by TOSOH CORPORATION)
Eluent: 0.2 M Phosphate buffer/CH₃CN = 9/1
Flow amount: 1.0 mL/min
Column temperature: 40°C
Detection: differential refractive index detector (RI)
Sample size: 0.5 mg/mL
Reference material: in terms of polyethylene glycol

The content of the dispersing agent is preferably 0.005 part by mass or more, more preferably 0.05 part by mass or more, and even more preferably 0.1 part by mass or more and also preferably 2.5 parts by mass or less, more preferably 1.0 part by mass or less, and more preferably 0.5 part by mass or less relative to 100 parts by mass of a hydraulic powder from the viewpoint of improving the fluidity of a hydraulic composition and suppressing delayed hardening of a hydraulic composition.

The hydraulic composition of the present disclosure may further contain other components and examples thereof include an AE agent, a retardant, a foaming agent, a thickening agent, a blowing agent, a waterproofing agent, and a superplasticizer.

The hydraulic composition of the present disclosure may be concrete or mortar. The hydraulic composition of the present disclosure is useful for use in any field, including use for self-leveling, use for fire resistance, use for plaster, use for light-weight concrete or heavyweight concrete, use for AE, use for repairs, use for prepacked, use for tremie, use for foundation improvement, use for grout, and use in cold weather. From the viewpoint of exhibiting strength at level of about 24 hours after the preparation of a hydraulic composition even with lowered heat curing energy and enabling demolding from a formwork at early term, it is preferably used for a concrete product such as a concrete vibration product and a centrifugally molded product.

The present disclosure provides a method for preparing a cured product including steps of producing a hydraulic composition containing the strength improver composition for a hydraulic powder of the present disclosure, and preferably the hydraulic composition of the present dislcosure, filling the produced hydraulic composition in a formwork followed by hardening and curing, and demolding the cured hydraulic composition. The product obtained by preparation of a hydraulic composition is preferably the hydraulic composition of the present disclosure.

According to the step of producing a hydraulic composition, preparation of a hydraulic composition is conducted by mixing the strength improver composition for a hydraulic powder of the present disclosure, with a hydraulic powder such as cement, an aggregate, and water. It may be also conducted by mixing them with a dispersing agent. From the viewpoint of smooth mixing of the strength improver composition for a hydraulic powder of the present disclosure and a hydraulic powder such as cement, the strength improver composition for a hydraulic powder of the present disclosure is preferably admixed in advance with water, or the strength improver composition for a hydraulic powder of the present disclosure, water, and a dispersing agent are admixed in advance, followed by mixing with cement. The strength improver composition for a hydraulic powder of the present disclosure can be also used as a composition containing water. Mixing of a hydraulic powder with water (preferably a mixed product of the strength improver composition for a hydraulic powder of the present disclosure and water, or a mixed product of the strength improver composition for a hydraulic powder of the present disclosure, a dispersing agent, and water) can be conducted by using a mixer such as a mortar mixer and a turbo two-shaft mixer. Furthermore, the mixing is conducted preferably for 1 minute or longer and more preferably 2 minutes or longer, and also preferably 5 minutes or shorter and more preferably 3 minutes or shorter. For preparing the hydraulic composition, it is possible that the materials, chemicals, and those in the amount described for the hydraulic composition can be used.

In the step of filling a formwork with the hydraulic composition followed by curing and hardening the composition, the formwork is filled with the resulting hydraulic composition followed by curing the composition. Examples of the formwork include a formwork for buildings and a formwork for concrete products. Examples of the method of filling a formwork with the hydraulic composition include a method of directly charging the composition from a mixer and a method of feeding the hydraulic composition under pressure with a pump and introducing the composition into the formwork.

With regard to the production of a cured product of a hydraulic composition, heat curing may be conducted for promoting hardening during curing of a hydraulic composition, and thus promoting the curing. Herein, according to the heat curing, the hydraulic composition is kept at a temperature of 40°C or more and 80°C or less to promote the hardening.

Examples of the cured product of a hydraulic composition using a formwork which is a concrete product include various block products for seashore protection, box culvert products, segment products used for tunneling work or the like, and molding products for a bridge pier as an civil engineering product, and curtain wall products, columns, crossbeams, and constructional members used for floor or the like as a constructional product.

For the use of the strength improver composition for a hydraulic powder of the present disclosure for improving the strength of a cured product of a hydraulic composition, the strength improver composition for a hydraulic powder is contained in a hydraulic composition to improve the strength of a cured product. As for the method for addition, there is a method of adding the strength improver composition for a hydraulic powder during production of a hydraulic composition, a method of adding it to a hydraulic compound during production of a hydraulic powder, or a method of adding it in advance to a hydraulic powder.

In the present specification, the matters described in the section of the strength improver composition for a hydraulic powder and the hydraulic composition can be also applied to a method for improving the strength of a cured product of a hydraulic composition of the present disclosure, a method for producing the hydraulic powder of the present invention, the hydraulic composition of the present disclosure, and the strength improver composition for a hydraulic powder of the present disclosure and use per the invention of the strength improver composition for a hydraulic powder of the present disclosure for improving the strength of a cured product of a hydraulic composition. In that case, "content" of the strength improver composition for a hydraulic powder of the present disclosure and dispersing agent in the section of the hydraulic composition can be translated as "blending amount" or "addition amount".

Embodiments of the strength improver composition for a hydraulic powder of the present disclosure are exemplified hereinbelow.

### Examples

The following Examples will describe the implementation of the present invention.

### (1) Strength improver composition

The reaction product obtained from the following Production Examples A1 to A10 is used alone or admixed with a component selected from a urea-formaldehyde condensate and hydroxymethane sulfonate [the component (B)], and 1 mole EO adduct of glycerin [the component (C)] and used as a strength improver composition of the present invention (in some Examples, it is described as "additive"). All strength improver compositions were in a liquid form. When the reaction product is used alone, the reaction product was used as it is unless specifically described otherwise. When a component selected from a urea-formaldehyde condensate and hydroxymethane sulfonate [the component (B)] is mixed, the reaction product [the component (A)] and the component (C) (in some Examples) were as it is admixed with the component (B) to obtain a strength improver composition. Furthermore, when the component (B) is used alone in Comparative Examples, the component (B) was as it is regarded as a strength improver composition. The solid matter in the compositions of Examples and Comparative Examples was a component selected from the component (A) (including unreacted one), the component (B), and the component (C).

### <Production Example>

### Production Example A1

0.41 g of sulfuric acid, and 25 g of paraformaldehyde were fed to a glass reaction vessel (200 ml flask) equipped with a stirrer, 76.74 g of glycerin, and the temperature was increased to 100°C under stirring. After increasing the temperature, stirring was conducted for 4 hours at the same temperature while moisture was extracted to the outside of the system. Then, the heating was terminated and the temperature was lowered to 50°C or lower by natural cooling followed by neutralization to pH 6 by using 48% by mass aqueous solution of sodium hydroxide to obtain the reaction product A1. The unreacted component (a1) accounted for 6.8% by mass in the solid matter according to quantitative gas chromatography analysis. Furthermore, the solid matter in the reaction product A1 accounted for 99% by mass. Meanwhile, the solid matter corresponds to a component other than water. The amount of water was measured by Karl Fischer method, and the content of the solid matter was determined from that amount.

### Production Example A2

0.41 g of sulfuric acid, and 67.57 g of aqueous solution of formaldehyde (37% by mass) were fed to a glass reaction vessel (200 ml flask) equipped with a stirrer, 76.74 g of glycerin, and the temperature was increased to 100°C under stirring. After increasing the temperature, stirring was conducted for 4 hours at the same temperature while moisture was extracted to the outside of the system. Then, the heating was terminated and the temperature was lowered to 50°C or lower by natural cooling followed by neutralization to pH 6 by using 48% by mass aqueous solution of sodium hydroxide to obtain the reaction product A2. The unreacted component (a1) accounted for 9.8% by mass in the solid matter according to quantitative gas chromatography analysis. Furthermore, the solid matter in the reaction product A2 accounted for 99% by mass.

### Production Example A3

76.74 g of glycerin, 0.41 g of sulfuric acid, and 67.57 g of aqueous solution of formaldehyde (37% by mass) were fed to a glass reaction vessel (200 ml flask) equipped with a stirrer and a condenser, and the temperature was increased to 80°C under stirring. After increasing the temperature, stirring was conducted for 4 hours at the same temperature while refluxing the moisture. Then, the heating was terminated and the temperature was lowered to 50°C or lower by natural cooling followed by neutralization to pH 6 by using 48% by mass aqueous solution of sodium hydroxide to obtain the reaction product A3. The unreacted component (a1) accounted for 21% by mass in the solid matter according to quantitative gas chromatography analysis. Furthermore, the solid matter in the reaction product A3 accounted for 69% by mass.

### Production Example A4

76.74 g of glycerin, 0.41 g of sulfuric acid, and 67.57 g of aqueous solution of formaldehyde (37% by mass) were fed to a glass reaction vessel (200 ml flask) equipped with a stirrer and a condenser, and the temperature was increased to 50°C under stirring. After increasing the temperature, stirring was conducted for 4 hours at the same temperature while refluxing the moisture. Then, the heating was terminated followed by neutralization to pH 6 by using 48% by mass aqueous solution of sodium hydroxide to obtain the reaction product A4. The unreacted component (a1) accounted for 30% by mass in the solid matter according to quantitative gas chromatography analysis. Furthermore, the solid matter in the reaction product A4 accounted for 72% by mass.

### Production Example A5

67.57 g of aqueous solution of formaldehyde (37% by mass) were fed to a glass reaction vessel (200 ml flask) equipped with a stirrer and a condenser, 76.74 g of glycerin, 0.41 g of sulfuric acid, and the stirring was conducted for 4 hours at 20°C while refluxing the moisture. Then, neutralization to pH 6 was conducted by using 48% by mass aqueous solution of sodium hydroxide to obtain the reaction product A5. The unreacted component (a1) accounted for 34% by mass in the solid matter according to quantitative gas chromatography analysis. Furthermore, the solid matter in the reaction product A5 accounted for 74% by mass.

### Production Example A6

76.74 g of glycerin, 0.204 g of sulfuric acid, and 33.78 g of aqueous solution of formaldehyde (37% by mass) were fed to a glass reaction vessel (200 ml flask) equipped with a stirrer and a condenser, and the temperature was increased to 80°C under stirring. After increasing the temperature, stirring was conducted for 4 hours at the same temperature while refluxing the moisture. Then, the heating was terminated and the temperature was lowered to 50°C or lower by natural cooling followed by neutralization to pH 6 by using 48% by mass aqueous solution of sodium hydroxide to obtain the reaction product A6. The unreacted component (a1) accounted for 42% by mass in the solid matter according to quantitative gas chromatography analysis. Furthermore, the solid matter in the reaction product A6 accounted for 78% by mass.

### Production Example A7

76.74 g of glycerin, 0.41 g of sulfuric acid, and 33.78 g of aqueous solution of formaldehyde (37% by mass) were fed to a glass reaction vessel (200 ml flask) equipped with a stirrer and a condenser, and the stirring was conducted for 4 hours at 20°C while refluxing the moisture. Then, neutralization to pH 6 was conducted by using 48% by mass aqueous solution of sodium hydroxide to obtain the reaction product A7. The unreacted component (a1) accounted for 57% by mass in the solid matter according to quantitative gas chromatography analysis. Furthermore, the solid matter in the reaction product A7 accounted for 83% by mass.

### Production Example A8

76.74 g of glycerin, 0.17 g of sodium hydroxide, and 67.57 g of aqueous solution of formaldehyde (37% by mass) were to a glass reaction vessel (200 ml flask) equipped with a stirrer and a condenser, and the temperature was increased to 80°C under stirring. After increasing the temperature, stirring was conducted for 4 hours at the same temperature while refluxing the moisture. Then, the heating was terminated and the temperature was lowered to 50°C or lower by natural cooling followed by neutralization to pH 6 by using sulfuric acid to obtain the reaction product A8. The unreacted component (a1) accounted for 34% by mass in the solid matter according to quantitative gas chromatography analysis. Furthermore, the solid matter in the reaction product A8 accounted for 75% by mass.

### Production Example A9

76.74 g of glycerin, 0.17 g of sodium hydroxide, and 67.57 g of aqueous solution of formaldehyde (37% by mass) were fed to a glass reaction vessel (200 ml flask) equipped with a stirrer and a condenser, and the stirring was conducted for 4 hours at 20°C while refluxing the moisture. Then, neutralization to pH 6 was conducted by using sulfuric acid to obtain the reaction product A9. The unreacted component (a1) accounted for 36% by mass in the solid matter according to quantitative gas chromatography analysis. Furthermore, the solid matter in the reaction product A9 accounted for 75% by mass.

### Production Example A10

113.46 g of an average 1 mole ethylene oxide adduct of glycerin, 0.41 g of sulfuric acid, and 25 g of paraformaldehyde were fed to a glass reaction vessel (200 ml flask) equipped with a stirrer, and the temperature was increased to 100°C under stirring. After increasing the temperature, stirring was conducted for 4 hours at the same temperature while moisture was extracted to the outside of the system. Then, the heating was terminated and the temperature was lowered to 50°C or lower by natural cooling followed by neutralization to pH 6 by using 48% by mass aqueous solution of sodium hydroxide to obtain the reaction product A10. The unreacted component (a1) accounted for 7.5% by mass in the solid matter according to quantitative gas chromatography analysis. Furthermore, the solid matter in the reaction product A10 accounted for 98% by mass.

Meanwhile, the reagents used in the above Production Examples are as described below. Furthermore, the reaction conditions or the like for the above Production Examples are summarized in Table 1. With regard to the description relating to moisture in the reaction system in Table 1, "Removed" indicates a reaction system in which moisture is removed to the outside of a system, and "Under reflux" indicates a reaction system which is conducted under reflux.
- Glycerin: 1,2,3-Propanetriol manufactured by Wako Pure Chemical Industries, Ltd.
- Adduct having on average 1 mole of ethylene oxide per glycerin: Glycerin (1,2,3-Propanetriol manufactured by Wako Pure Chemical Industries, Ltd.) added with 1 mole of ethylene oxide on average
- Sulfuric acid: Manufactured by Sigma-Aldrich
- Sodium hydroxide: Reagent with special grade, manufactured by Wako Pure Chemical Industries, Ltd.
- Paraformaldehyde: Wako first grade, manufactured by Wako Pure Chemical Industries, Ltd.
- Formaldehyde: Reagent with special grade, manufactured by Wako Pure Chemical Industries, Ltd.

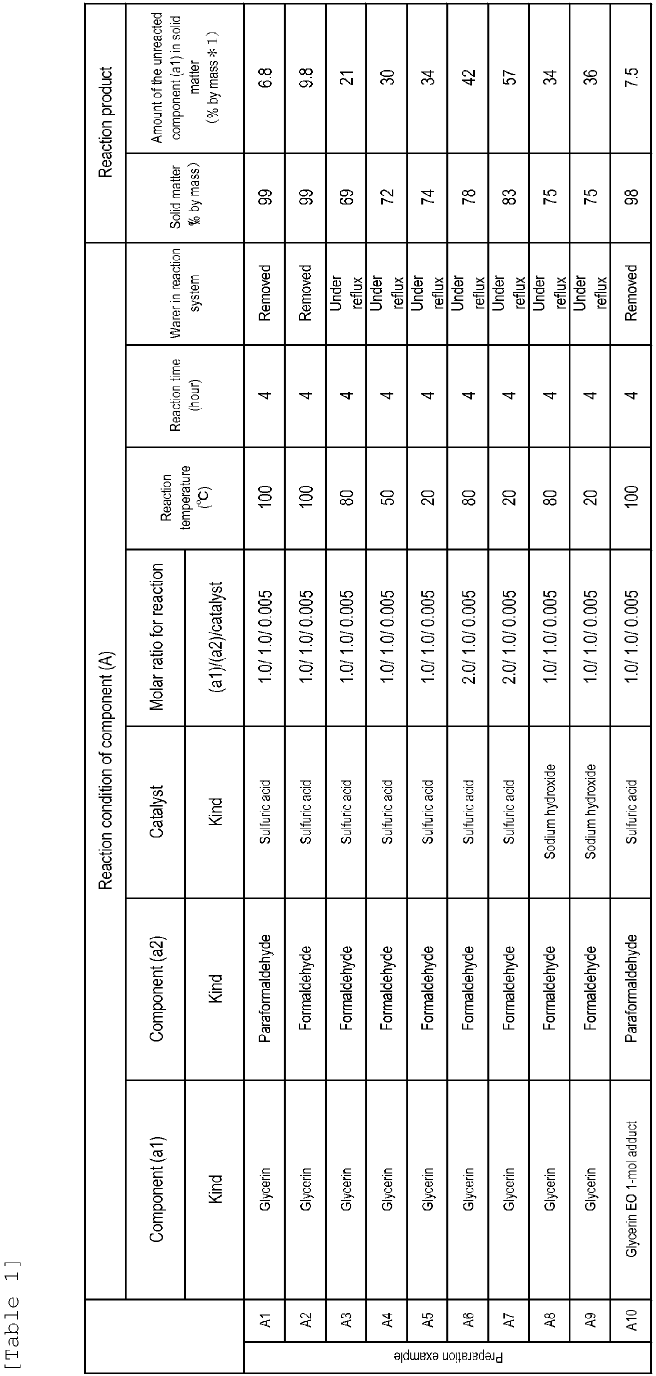

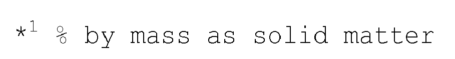

As for the urea-formaldehyde condensate, hydroxymethane sulfonate and the Polymer 1, those described below were used.
- Urea-formaldehyde condensate: obtained by the following synthetic method
   450 g of urea and 3588 g of distilled water fed to a three neck flask (volume of 10 liters) and mixed for 10 minutes at 200 rpm by using stirring wings. Thereafter, pH was adjusted to 11 and the temperature was increased to 40°C. Subsequently, 305 g of 37% by mass aqueous solution of formaldehyde (formalin) was added dropwise thereto over 90 minutes. After the dropwise addition was completed, stirring was continued for 1 hour for aging followed by cooling to room temperature to obtain a urea-formaldehyde condensate in which the content of the solid matter (urea-formaldehyde condensate) was 13.5% by mass.
- Sodium hydroxymethane sulfonic acid: manufactured by Wako Pure Chemical Industries, Ltd.
- Polymer 1: obtained by the following synthetic method.

114 g of water was fed to a glass reaction vessel (four neck flask) equipped with a stirrer, and nitrogen substitution was conducted under stirring. The temperature was increased to 80°C in nitrogen atmosphere Each of an aqueous solution in which 300 g of an aqueous solution of 60% by mass ω-methoxypolyethylene glycol monomethacrylate (average added mole number of ethylene oxide was 120; ester purity was 100% by mass), 11.5 g of methacrylic acid (reagent: manufactured by Wako Pure Chemical Industries, Ltd.) and 1.2 g of 3-mercaptopropionic acid are mixed and dissolved, and an aqueous solution in which 1.9 g of ammonium persulfate is dissolved in 45 g of water was added dropwise to the above reaction vessel over 1.5 hours. After that, it was aged for 1 hour at 80°C and further added dropwise with an aqueous solution in which 0.8 g of ammonium persulfate is dissolved in 15 g of water over 30 minutes followed by aging for 1.5 hours at 80°C. Once the aging is completed, it was cooled to 40°C or lower followed by neutralization with 9.6 g of 48% by mass aqueous solution of sodium hydroxide to obtain a copolymer with a weight average molecular weight of 54000 (Polymer 1) (neutralization degree of 0.7). After that, the solid matter was adjusted to 40% by mass by using water to obtain 40% by mass aqueous solution of the Polymer 1. The Polymer 1 is a polycarboxylic acid copolymer in which the molar ratio of the monomer (1)/the monomer (2) is 20/80.

### (2) Preparation of hydraulic powder

### (2-1) Preparation of hydraulic powder having admixture content of 0% by mass

A pulverization material containing 95% by mass of clinker and 5% by mass of dihydrate gypsum was pulverized by a ball mill to a Blaine value of 3600 cm²/g without adding a pulverization aid. As a result, a hydraulic powder having an admixture content of 0% by mass was produced.

### (2-2) Preparation of hydraulic powder having admixture content of 5% by mass

A pulverization material containing 90% by mass of clinker, 5% by mass of dihydrate gypsum, and 5% by mass of blast furnace slag was pulverized by a ball mill to a Blaine value of 3600 cm²/g without adding a pulverization aid. As a result, a hydraulic powder having an admixture content of 5% by mass was produced.

### (2-3) Preparation of hydraulic powder having admixture content of 10% by mass

A pulverization material containing 86% by mass of clinker, 4% by mass of dihydrate gypsum, 5% by mass of blast furnace slag, and 5% by mass of fly ash was pulverized by a ball mill to a Blaine value of 3600 cm²/g without adding a pulverization aid. As a result, a hydraulic powder having an admixture content of 10% by mass was produced.

### (2-4) Preparation of hydraulic powder having admixture content of 30% by mass

A pulverization material containing 67% by mass of clinker, 3% by mass of dihydrate gypsum, 15% by mass of blast furnace slag, and 15% by mass of fly ash was pulverized by a ball mill to a Blaine value of 3600 cm²/g without adding a pulverization aid. As a result, a hydraulic powder having an admixture content of 30% by mass was produced.

### (2-5) Preparation of hydraulic powder having admixture content of 47% by mass

A pulverization material containing 50% by mass of clinker, 3% by mass of dihydrate gypsum, 25% by mass of blast furnace slag, and 22% by mass of fly ash was pulverized by a ball mill to a Blaine value of 3600 cm²/g without adding a pulverization aid. As a result, a hydraulic powder having an admixture content of 47% by mass was produced.

### (2-6) Preparation of hydraulic powder having admixture content of 70% by mass

A pulverization material containing 28% by mass of clinker, 2% by mass of dihydrate gypsum, 35% by mass of blast furnace slag, and 35% by mass of fly ash was pulverized by a ball mill to a Blaine value of 3600 cm²/g without adding a pulverization aid. As a result, a hydraulic powder having an admixture content of 70% by mass was produced.

### (2-7) Materials used

- Clinker: clinker for ordinary Portland cement (cement passed through a 3.5-mm sieve, described as C in the table), which is obtained by calcination of combination of limestone, clay, silica, iron oxide material, or the like to have components as follows; CaO: about 65%, SiO₂: about 22%, Al₂O₃: about 5%, Fe₂O₃: about 3%, MgO and others: about 3% (on the basis of mass) followed by primary pulverization using a crusher and a grinder.
- Dihydrate gypsum: special grade reagent, manufactured by Wako Pure Chemical Industries, Ltd. (described as G in the table)
- Blast furnace slag: obtained by primary pulverization of blast furnace slag using a crusher and a grinder (slag passed through a 3.5-mm sieve, and described as Slag in the table)
- Fly ash: commercially available product manufactured by Chubu Electric Power Co., Inc. (described as FA in the table)

### (2-8) Ball mill

As a ball mill, AXB-15 manufactured by Seiwa Giken Co., Ltd. was used. The volume of the stainless pot was 18 liters (outer diameter of 300 mm), and 70 stainless balls having 30 mmφ (nominal diameter: 1·3/16) and 70 balls having 20 mmφ (nominal diameter: 3/4) were used (140 balls in total). The rotational speed of the ball mill was 45 rpm. Furthermore, the time for discharging the pulverized product during pulverization was set to 1 minute.

### (2-9) Measurement of Blaine value

For the measurement of a Blaine value, the Blaine air permeability apparatus defined in the physical test method for cement (JIS R 5201) was used.

### (2-10) Measurement of BET specific surface area

The measurement of a BET specific surface area was conducted under the following conditions by using Macsorb HM-model 1201 (manufactured by Mountech).
- Deaeration: 100°C × 30 minutes, cooling × 4 minutes
- Gas for measurement: as a carrier gas, helium was used, and nitrogen was used as a coolant and an adsorbing material. Furthermore, the mixed gas concentration was 30.4% and the flow amount was 25 ml/min.

### (3) Preparation of hydraulic composition

Hydraulic compositions were prepared in accordance with Physical testing methods for cement (JIS R 5201), Annex 2 (Testing methods for cement - Determination of strength). Note that, with respect to materials used, the hydraulic powders prepared in (2) were used as cement, and the strength improver compositions for hydraulic powders were added to kneading water (except for some Examples and Comparative Examples).

### (4) Compressive strength test

The test was conducted in accordance with Physical testing methods for cement (JIS R 5201), Annex 2 (Testing methods for cement - Determination of strength). Each measured value and each relative value having the measured value of 100 for Comparative Example in which a strength improver composition (an additive) is not added (for the Test Examples, it indicates Comparative Example for the same Test Example) are described in the tables.

### Examples 1-1 to 1-4 and Comparative Examples 1-1 to 1-5

In the following, Examples 1-1 and 1-4 are provided for reference purposes. Examples 1-2 and 1-3 are according to the invention.

By using the additive described in Table 2 in an amount described in Table 2 with relative to 100 parts by mass of a hydraulic powder having an admixture content of 0% by mass, a hydraulic composition was prepared. Then, the compressive strength was measured after 3 days or after 28 days from the preparation of the hydraulic composition. Meanwhile, all the addition amounts described in the table are expressed in part by mass based on solid matter (the same shall apply hereinbelow).

Meanwhile, for Comparative Example 1-4, glycerin and formaldehyde were separately added to mixing water and mortar was produced immediately thereafter [Glycerin/Formaldehyde = 75/25 (mass ratio)]. The addition amount of the additive used for Comparative Example 1-4 was set to be the same as the raw material feeding ratio of the reaction products A1, A5 and A9 and the total amount was set to be equal to the amount of Example (0.015 part by mass relative to 100 parts by mass of hydraulic powder). The results are described in Table 2.

**[Table 2]**

| Composition of hydraulic powder | | Additive | | Compressive strength (N/mm²) | | | |
|---|---|---|---|---|---|---|---|
| | | | | Measured value | | Relative value | |
| C:95% | | Kind | Added amount relative to 100 parts by mass of hydraulic powder parts by mass) | After 3 days | After 28 days | After 3 days | After 28 days |
| G:5% | | | | | | | |
| Comparative example | 1-1 | - | - | 21.2 | 41.5 | 100 | 100 |
| | 1-2 | Triethanol amine | 0.015 | 22.1 | 40.8 | 104 | 98 |
| Example | 1-1 | Reaction product A1 | 0.015 | 22.3 | 43.7 | 105 | 105 |
| | 1-2 | Reaction product A5 | 0.015 | 24.3 | 44.3 | 115 | 107 |
| | 1-3 | Reaction product A9 | 0.015 | 24.1 | 45.8 | 114 | 116 |
| | 1-4 | Reaction product A10 | 0.015 | 22.3 | 43.1 | 105 | 104 |
| Comparative example | 1-3 | Glycerin | 0.0113 | 21.4 | 41.6 | 101 | 100 |
| | 1-4 | Glycerin | 0.0113 | 21.5 | 41.8 | 101 | 101 |
| | | Formaldehyde | 0.0037 | | | | |
| | 1-5 | Formaldehyde | 0.0037 | 21.5 | 41.8 | 101 | 101 |

### Examples 2-1 to 2-16 and Comparative Examples 2-1 to 2-5

In the following, Examples 2-1 and 2-16 are provided for reference purposes. Examples 2-2 to 2-15 are according to the invention.

By using the additive described in Table 3 in an amount described in Table 3 with relative to 100 parts by mass of a hydraulic powder having an admixture content of 47% by mass, a hydraulic composition was prepared. Then, the compressive strength was measured after 3 days or after 28 days from preparation of the hydraulic composition.

Meanwhile, for Comparative Example 2-4, glycerin and formaldehyde were separately added to mixing water and mortar was produced immediately thereafter [Glycerin/Formaldehyde = 75/25 (mass ratio)]. The addition amount of the additive used for Comparative Example 2-4 was set to be the same as the raw material feeding ratio of the reaction products A1, A5 and A9 and the total amount was set to be equal to the amount of Example 2-1 and the like (0.015 part by mass relative to 100 parts by mass of hydraulic powder). The results are described in Table 3.

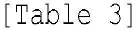

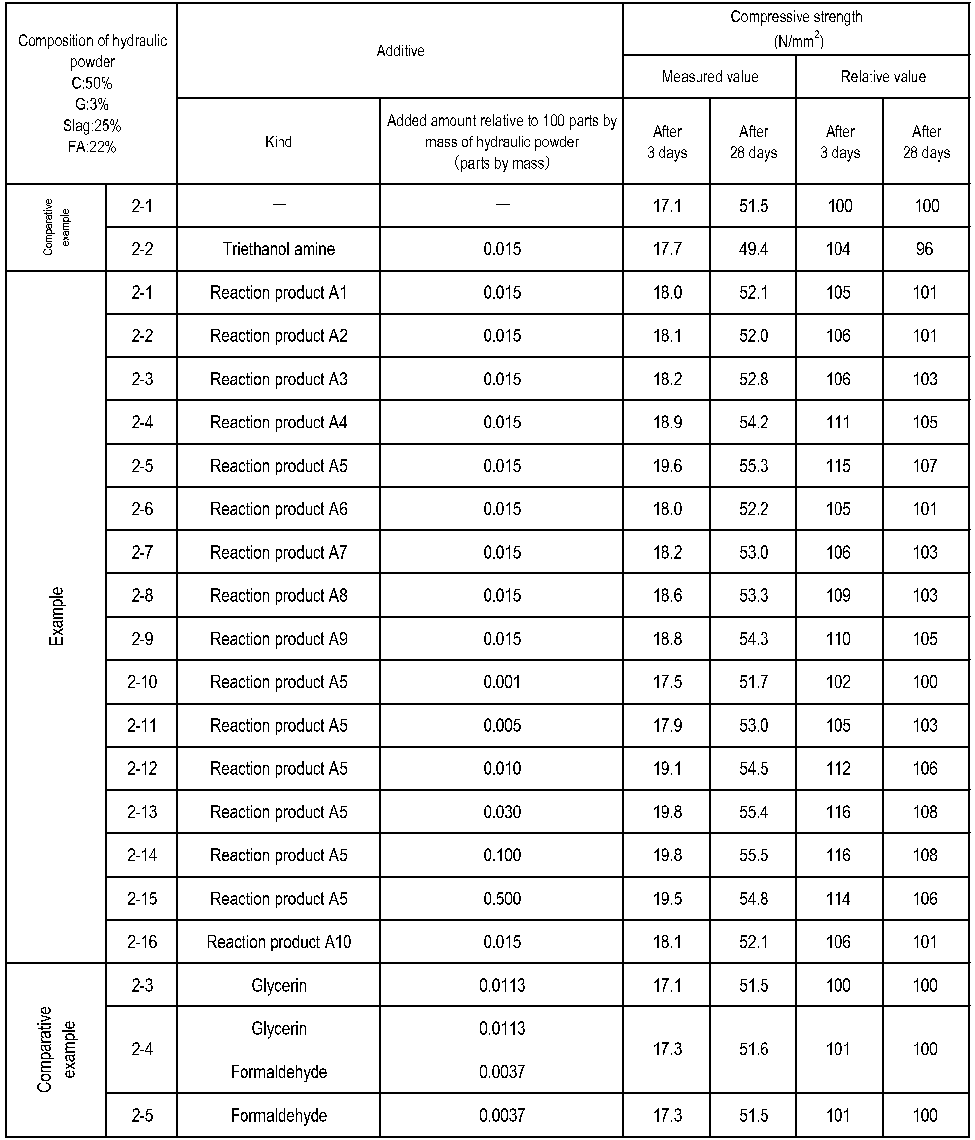

### Examples 3-1 to 3-13 and Comparative Examples 3-1 to 3-6

In the following, Examples 3-1 to 3-6, and 3-11 are provided for reference purposes. Examples 3-7 to 3-10 and 3-12 and 3-13 are according to the invention.

By using the strength improver compositions described in Table 4 in an amount described in Table 4 with relative to 100 parts by mass of a hydraulic powder having an admixture content of 47% by mass, a hydraulic composition was prepared. Then, the compressive strength was measured after 3 days or after 28 days from preparation of the hydraulic composition. The results are described in Table 4.

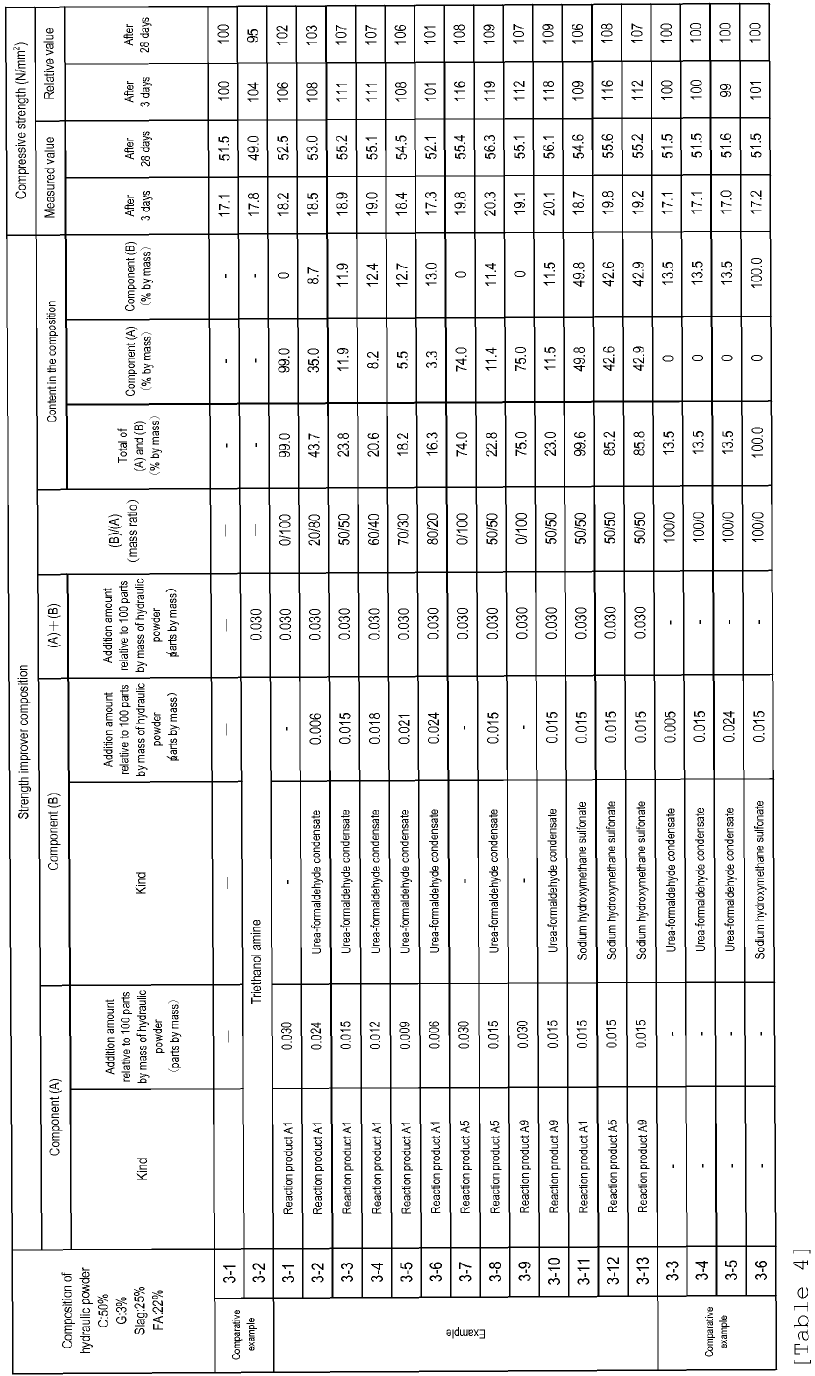

In Table 4, for a case in which only the component (A) is used, % by mass of the component (A) in "Content in the composition" indicates % by mass of the solid matter in the reaction product (A).

### Test Examples 4 to 9

For a case in which the reaction product A1 and urea-formaldehyde condensate are used in combination as a strength improver composition (examples provided for reference purposes with sub-numbering of "-1") and a case in which only the reaction product A5 is used (examples of the invention with sub-numbering of "-2") in a hydraulic powder having different admixture content, the compressive strength was measured after 3 days or after 28 days from production of the hydraulic composition. The results are described in Table 5. Meanwhile, in Examples 4-1, 5-1, 6-1, 7-1, 8-1 and 9-1, the strength improver composition was prepared in the same manner as Example 3-3. The total content of the component (A) and the component (B) in those compositions was 23.8% by mass.

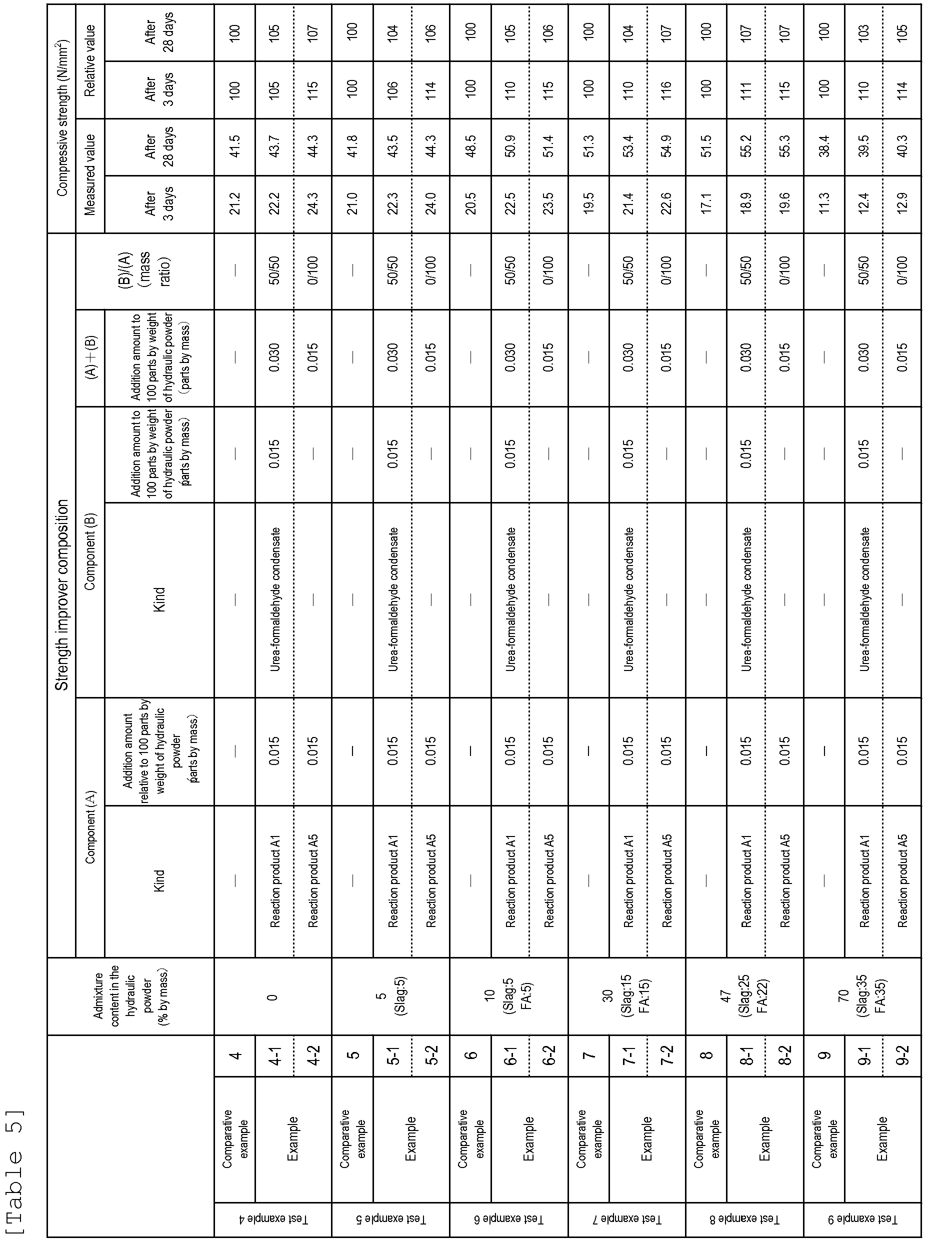

### Examples 10-1 to 10-3 and Comparative Example 10

In the following, Example 10-1 is provided for reference purposes. Examples 10-2 and 10-3 are according to the invention.

The pulverization property was measured for a case in which the strength improver composition was used as an additive composition for pulverization, and the compressive strength was measured after 3 days or after 28 days from production of the hydraulic composition.

As a hydraulic compound, a composition of a hydraulic powder having an admixture content of 47% by mass was used. As an additive composition for pulverization, a combined use of the reaction product A1 and urea-formaldehyde condensate, use of the reaction product A5 alone, and a combined use of the reaction product A5, a urea-formaldehyde condensate [the component (B)], and an average 1 mole ethylene oxide adduct of glycerin [the component (C), in the table, it is designated as glycerin EO 1-mol adduct of glycerin] were used. Herein, the average 1 mole ethylene oxide adduct of glycerin indicates that ethylene oxide was added at 1 mole on average to glycerin (1,2,3-propanetriol manufactured by Wako Pure Chemical Industries, Ltd.).

The components of Table 6 were added in an amount described in Table 6 to 100 parts by mass of a hydraulic compound followed by pulverization of the hydraulic compound using a ball mill. The pulverization property was then evaluated by using a BET specific surface area after pulverization for 38 minutes as an indicator. Furthermore, a hydraulic composition was produced by using the obtained hydraulic powder, and the compressive strength was measured after 3 days or after 28 days from the production of the hydraulic composition. The results are described in Table 6. Meanwhile, for Examples 10-1 and 10-3, the strength improver composition was prepared in the same manner as Example 3-3. At that time, in Example 10-3, a predetermined amount of the component (C) was also admixed and the solid matter concentration was adjusted with water. The total content of the component (A) and the component (B) in the composition of Example 10-1 was 23.8% by mass. Furthermore, the total content of the component (A), the component (B), and the component (C) in the composition of Example 10-3 was 37.2% by mass.

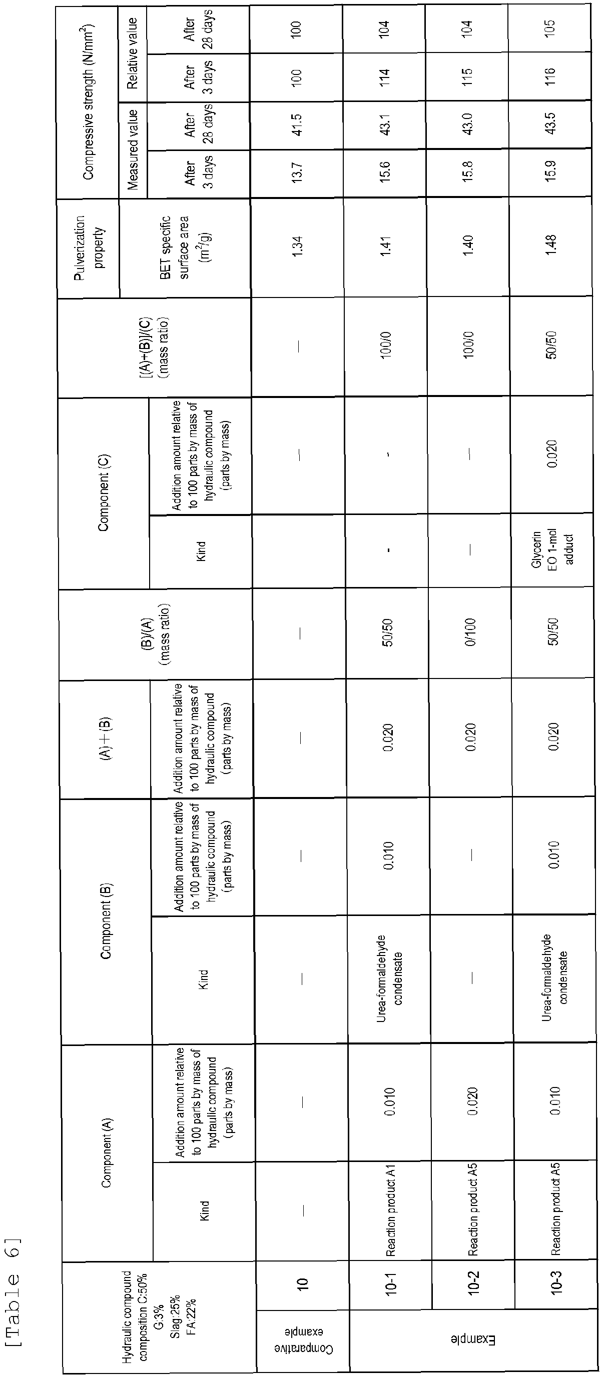

### Examples 11-1 to 11-2 and Comparative example 11

In the following, Example 11-1 is provided for reference purposes. Example 11-2 is according to the invention.

The compressive strength was measured after 3 days or after 28 days from the production of the hydraulic composition in a case in which a strength improver composition is used in combination with a dispersing agent. The hydraulic composition was produced by using the dispersing agent and strength improver composition described in Table 7, each in the amount described in Table 7, in 100 parts by mass of a hydraulic powder having an admixture content of 47% by mass.

As a strength improver composition, a combined use of the reaction product A1 and a urea-formaldehyde condensate or single use of the reaction product A5 was employed. As a dispersing agent, a polycarboxylic acid dispersing agent (Polymer 1) was used.

Meanwhile, in Comparative Example 11 and Examples 11-1 and 11-2, the test was conducted after changing the water amount of 225 g [mass ratio of water/hydraulic powder is 50 (%)] of other Examples and Comparative Examples to the water amount of 180 g [mass ratio of water/hydraulic powder is 40 (%)]. In Examples 11-1 and 11-2, a chemical admixture was prepared by mixing the Polymer 1 with a strength improver composition. The resulting chemical admixture was then added to mixing water.

The compressive strength was measured after 3 days and after 28 days from the production of the hydraulic composition. The results are described in Table 7. Meanwhile, in Example 11-1, the strength improver composition was prepared in the same manner as Example 3-3. The total content of the component (A) and the component (B) in the composition was 23.8% by mass.

### [Table 7]

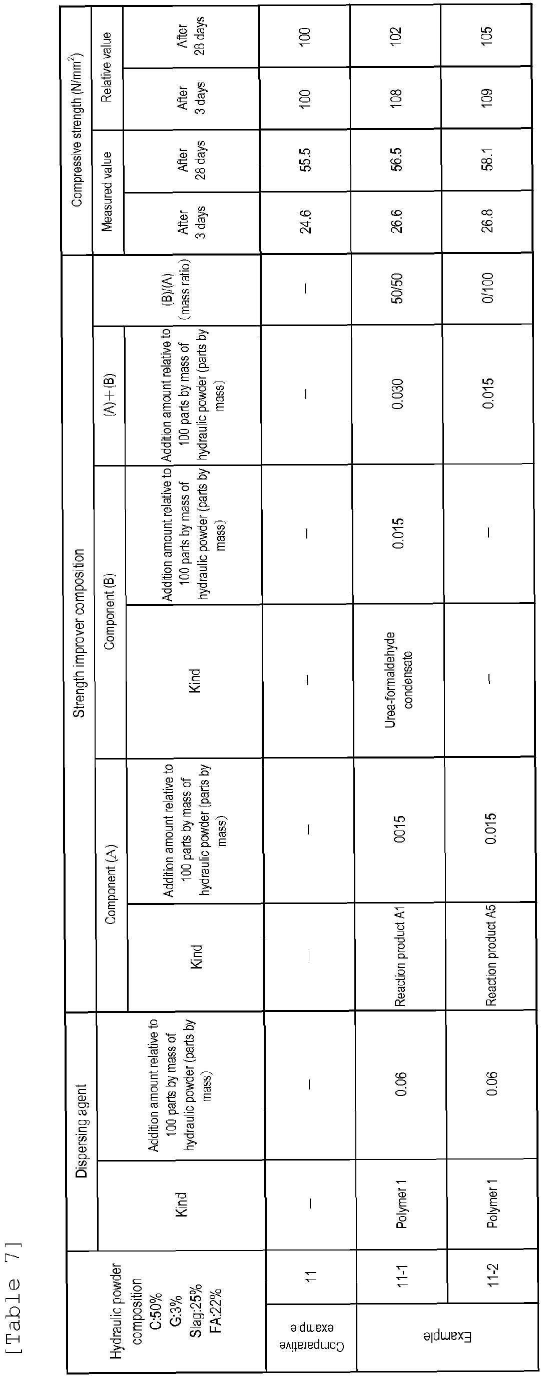

## Claims

1. A method for producing a hydraulic powder, comprising adding, during pulverization of a hydraulic compound, a strength improver composition for a hydraulic powder which contains a reaction product [hereinbelow, referred to as the component (A)] obtained by reacting (a1) glycerin [hereinbelow, referred to as the component (al)] and (a2) formaldehyde [hereinbelow, referred to as the component (a2)], at, in terms of solid matter, 0.0005 part by mass or more and 1.0 part by mass or less relative to 100 parts by mass of the hydraulic compound.

2. The method for producing a hydraulic powder according to claim 1, wherein the strength improver composition for a hydraulic powder further contains at least one compound selected from the group consisting of a urea-formaldehyde condensate and hydroxymethane sulfonate [hereinbelow, referred to as the component (B)].

3. The method for producing a hydraulic powder according to claim 1 or 2, wherein the hydraulic compound comprises at least one substance selected from the group consisting of blast furnace slag, fly ash, and silica fume at 10% by mass or more and 80% by mass or less.

4. The method for producing a hydraulic powder according to claim 2 or 3, wherein the mass ratio of the component (B) to the component (A), in terms of (B)/(A), is more than 0/100 and 80/20 or less in the strength improver composition for a hydraulic powder.

5. The method for producing a hydraulic powder according to any one of claims 1 to 4, wherein a reaction system for obtaining the component (A) of the strength improver composition for a hydraulic powder from the component (a1) and the component (a2) is for removing moisture from the system at a reaction temperature of 80°C or higher.

6. The method for producing a hydraulic powder according to any one of claims 1 to 4, wherein a reaction system for obtaining the component (A) of the strength improver composition for a hydraulic powder from the component (a1) and the component (a2) is a reaction system in which reflux of moisture is conducted and the reaction temperature is 80°C or lower.

7. The method for producing a hydraulic powder according to any one of claims 1 to 6, wherein at least one compound selected from the group consisting of an ethylene oxide adduct of glycerin, diethylene glycol, and triethanolamine is contained during pulverization of the hydraulic compound.

8. An additive composition for pulverizing a hydraulic compound, comprising:
a strength improver composition for a hydraulic powder which contains a reaction product [hereinbelow, referred to as the component (A)] obtained by reacting (a1) glycerin [hereinbelow, referred to as the component (al)] and (a2) formaldehyde [hereinbelow, referred to as the component (a2)]; and
at least one compound selected from the group consisting of an ethylene oxide adduct of glycerin, diethylene glycol, and triethanolamine.

9. Use of a strength improver composition for a hydraulic powder as a pulverization aid during pulverization of a hydraulic compound, the strength improver composition comprising a reaction product [hereinbelow, referred to as the component (A)] obtained by reacting (a1) glycerin [hereinbelow, referred to as the component (al)] and (a2) formaldehyde [hereinbelow, referred to as the component (a2)],
wherein the strength improver composition is added at, in terms of solid matter, 0.0005 part by mass or more and 1.0 part by mass or less relative to 100 parts by mass of the hydraulic compound.

10. The use of a strength improver composition for a hydraulic powder as a pulverization aid according to claim 9, wherein the strength improver composition for a hydraulic powder further comprises at least one compound selected from the group consisting of a urea-formaldehyde condensate and hydroxymethane sulfonate [hereinbelow, referred to as the component (B)].

11. The use of a strength improver composition for a hydraulic powder as a pulverization aid according to claim 9 or 10, wherein the hydraulic compound comprises at least one substance selected from the group consisting of blast furnace slag, fly ash, and silica fume at 10% by mass or more and 80% by mass or less.

12. The use of a strength improver composition for a hydraulic powder as a pulverization aid according to claim 10 or 11, wherein the mass ratio of the component (B) to the component (A), in terms of (B)/(A), is more than 0/100 and 80/20 or less in the strength improver composition for a hydraulic powder.

13. The use of a strength improver composition for a hydraulic powder as a pulverization aid according to any one of claims 9 to 12, wherein a reaction system for obtaining the component (A) of the strength improver composition for a hydraulic powder from the component (a1) and the component (a2) is for removing moisture from the system at a reaction temperature of 80°C or higher.

14. The use of a strength improver composition for a hydraulic powder as a pulverization aid according to any one of claims 9 to 12, wherein a reaction system for obtaining the component (A) of the strength improver composition for a hydraulic powder from the component (a1) and the component (a2) is a reaction system in which reflux of moisture is conducted and the reaction temperature is 80°C or lower.

15. The use of a strength improver composition for a hydraulic powder as a pulverization aid according to any one of claims 9 to 14, wherein the strength improver composition is used as a pulverization aid for pulverizing the hydraulic compound in the presence of at least one compound selected from the group consisting of an ethylene oxide adduct of glycerin, diethylene glycol, and triethanolamine.

## Patentansprüche

1. Verfahren zur Herstellung eines hydraulischen Pulvers, umfassend, während der Pulverisierung einer hydraulischen Verbindung, die Zugabe einer festigkeitsverbessernden Zusammensetzung für ein hydraulisches Pulver, die ein Reaktionsprodukt [nachstehend als Komponente (A) bezeichnet], das durch Reaktion von (a1) Glycerin [nachstehend als Komponente (a1) bezeichnet] und (a2) Formaldehyd [nachstehend als Komponente (a2) bezeichnet] erhalten wird, in Bezug auf die festen Bestandteile in einer Menge von 0,0005 Gewichtsteilen oder mehr und 1,0 Gewichtsteilen oder weniger, bezogen auf 100 Gewichtsteile einer hydraulischen Verbindung, enthält.

2. Verfahren zur Herstellung eines hydraulischen Pulvers gemäß Anspruch 1, wobei die festigkeitsverbessernde Zusammensetzung für ein hydraulisches Pulver ferner mindestens eine Verbindung ausgewählt aus der Gruppe bestehend aus einem Harnstoff-Formaldehyd-Kondensat und Hydroxymethansulfonat [nachstehend als Komponente (B) bezeichnet] enthält.

3. Verfahren zur Herstellung eines hydraulischen Pulvers gemäß Anspruch 1 oder 2, wobei die hydraulische Verbindung mindestens eine Substanz ausgewählt aus der Gruppe bestehend aus Hochofenschlacke, Flugasche und Silikastaub, in einer Menge von 10 Massen-% oder mehr und 80 Massen-% oder weniger umfasst.

4. Verfahren zur Herstellung eines hydraulischen Pulvers gemäß Anspruch 2 oder 3, wobei das Massenverhältnis der Komponente (B) zu der Komponente (A), im Sinne von (B)/(A), in der festigkeitsverbessernden Zusammensetzung für ein hydraulisches Pulver mehr als 0/100 und 80/20 oder weniger beträgt.

5. Verfahren zur Herstellung eines hydraulischen Pulvers gemäß einem der Ansprüche 1 bis 4, wobei ein Reaktionssystem zur Gewinnung der Komponente (A) der festigkeitsverbessernden Zusammensetzung für ein hydraulisches Pulver aus der Komponente (a1) und der Komponente (a2) dazu dient, Feuchtigkeit bei einer Reaktionstemperatur von 80 °C oder höher aus dem System zu entfernen.

6. Verfahren zur Herstellung eines hydraulischen Pulvers gemäß einem der Ansprüche 1 bis 4, wobei ein Reaktionssystem zur Gewinnung der Komponente (A) der festigkeitsverbessernden Zusammensetzung für ein hydraulisches Pulver aus der Komponente (a1) und der Komponente (a2) ein Reaktionssystem ist, in welchem Feuchtigkeitsrückfluss durchgeführt wird und in dem die Reaktionstemperatur 80 °C oder weniger beträgt.

7. Verfahren zur Herstellung einer hydraulischen Zusammensetzung gemäß einem der Ansprüche 1 bis 6, wobei während des Pulverisierens der hydraulischen Verbindung mindestens eine Verbindung ausgewählt aus der Gruppe bestehend aus einem Ethylenoxidaddukt von Glycerin, Diethylenglykol und Triethanolamin enthalten ist.

8. Additivzusammensetzung für die Pulverisierung einer hydraulischen Verbindung, umfassend:
eine festigkeitsverbessernde Zusammensetzung für ein hydraulisches Pulver, das ein Reaktionsprodukt [nachstehend als Komponente (A) bezeichnet] enthält, das durch Reaktion von (a1) Glycerin [nachstehend als Komponente (a1) bezeichnet] und (a2) Formaldehyd [nachstehend als Komponente (a2) bezeichnet] erhalten wird; und
mindestens eine Verbindung ausgewählt aus der Gruppe bestehend aus einem Ethylenoxidaddukt von Glycerin, Diethylenglykol und Triethanolamin.

9. Verwendung einer festigkeitsverbessernden Zusammensetzung für ein hydraulisches Pulver als Pulverisierungshilfsmittel während des Pulverisierens einer hydraulischen Verbindung, wobei die festigkeitsverbessernde Zusammensetzung ein Reaktionsprodukt [nachstehend als Komponente (A) bezeichnet] umfasst, das durch Reaktion von (a1) Glycerin [nachstehend als Komponente (a1) bezeichnet] und (a2) Formaldehyd [nachstehend als Komponente (a2) bezeichnet] erhalten wird,
wobei die festigkeitsverbessernde Zusammensetzung in Bezug auf die festen Bestandteile, in einer Menge von 0,0005 Gewichtsteilen oder mehr und 1,0 Gewichtsteilen oder weniger bezogen auf 100 Gewichtsteile einer hydraulischen Verbindung hinzugefügt wird.

10. Verwendung einer festigkeitsverbessernden Zusammensetzung für ein hydraulisches Pulver als Pulverisierungshilfsmittel gemäß Anspruch 9, wobei die festigkeitsverbessernde Zusammensetzung für ein hydraulisches Pulver ferner mindestens eine Verbindung ausgewählt aus der Gruppe bestehend aus einem Harnstoff-Formaldehyd-Kondensat und Hydroxymethansulfonat [nachstehend als Komponente (B) bezeichnet] enthält.

11. Verwendung einer festigkeitsverbessernden Zusammensetzung für ein hydraulisches Pulver als Pulverisierungshilfsmittel gemäß Anspruch 9 oder 10, wobei die hydraulische Verbindung mindestens eine Substanz ausgewählt aus der Gruppe bestehend aus Hochofenschlacke, Flugasche und Silikastaub, in einer Menge von 10 Massen-% oder mehr und 80 Massen-% oder weniger umfasst.

12. Verwendung einer festigkeitsverbessernden Zusammensetzung für ein hydraulisches Pulver als Pulverisierungshilfsmittel gemäß Anspruch 10 oder 11, wobei das Massenverhältnis der Komponente (B) zu der Komponente (A), im Sinne von (B)/(A), in der festigkeitsverbessernden Zusammensetzung für ein hydraulisches Pulver mehr als 0/100 und 80/20 oder weniger beträgt.

13. Verwendung einer festigkeitsverbessernden Zusammensetzung für ein hydraulisches Pulver als Pulverisierungshilfsmittel gemäß einem der Ansprüche 9 bis 12, wobei ein Reaktionssystem zur Gewinnung der Komponente (A) der festigkeitsverbessernden Zusammensetzung für ein hydraulisches Pulver aus der Komponente (a1) und der Komponente (a2) dazu dient, Feuchtigkeit bei einer Reaktionstemperatur von 80 °C oder höher aus dem System zu entfernen.

14. Verwendung einer festigkeitsverbessernden Zusammensetzung für ein hydraulisches Pulver als Pulverisierungshilfsmittel gemäß einem der Ansprüche 9 bis 12, wobei ein Reaktionssystem zur Gewinnung der Komponente (A) der festigkeitsverbessernden Zusammensetzung für ein hydraulisches Pulver aus der Komponente (a1) und der Komponente (a2) ein Reaktionssystem ist, in welchem Feuchtigkeitsrückfluss durchgeführt wird und in dem die Reaktionstemperatur 80 °C oder weniger beträgt.

15. Verwendung einer festigkeitsverbessernden Zusammensetzung für ein hydraulisches Pulver als Pulverisierungshilfsmittel gemäß einem der Ansprüche 9 bis 14, wobei die festigkeitsverbessernde Zusammensetzung als Pulverisierungshilfsmittel zur Pulverisierung der hydraulischen Verbindung in Gegenwart mindestens einer Verbindung ausgewählt aus der Gruppe bestehend aus einem Ethylenoxidaddukt von Glycerin, Diethylenglykol und Triethanolamin verwendet wird.

## Revendications

1. Procédé de production d'une poudre hydraulique, comprenant l'ajout, lors de la pulvérisation d'un composé hydraulique, d'une composition d'agent d'amélioration de résistance pour une poudre hydraulique qui contient un produit réactionnel [ci-dessous dans le présent document, désigné par composant (A)] obtenu par la réaction (a1) de glycérine [ci-dessous dans le présent document, désignée par composant (al)] et (a2) de formaldéhyde [ci-dessous dans le présent document, désigné par composant (a2)], à, en termes de matière solide, 0,0005 partie en masse ou plus et 1,0 partie en masse ou moins par rapport à 100 parties en masse du composé hydraulique.

2. Procédé de production d'une poudre hydraulique selon la revendication 1, dans lequel la composition d'agent d'amélioration de résistance pour une poudre hydraulique contient en outre au moins un composé sélectionné dans le groupe consistant en un condensat d'urée-formaldéhyde et un hydroxyméthane sulfonate [ci-dessous dans le présent document, désigné par composant (B)].

3. Procédé de production d'une poudre hydraulique selon la revendication 1 ou 2, dans lequel le composé hydraulique comprend au moins une substance sélectionnée dans le groupe consistant en un laitier de haut fourneau, une cendre volante, et une fumée de silice à 10 % en masse ou plus et 80 % en masse ou moins.

4. Procédé de production d'une poudre hydraulique selon la revendication 2 ou 3, dans lequel le rapport en masse entre le composant (B) et le composant (A), en termes de (B)/(A), est de plus de 0/100 et de 80/20 ou moins dans la composition d'agent d'amélioration de résistance pour une poudre hydraulique.

5. Procédé de production d'une poudre hydraulique selon l'une quelconque des revendications 1 à 4, dans lequel un système réactionnel pour obtenir le composant (A) de la composition d'agent d'amélioration de résistance pour une poudre hydraulique à partir du composant (a1) et du composant (a2) est pour éliminer l'humidité du système à une température réactionnelle de 80 °C ou plus.

6. Procédé de production d'une poudre hydraulique selon l'une quelconque des revendications 1 à 4, dans lequel un système réactionnel pour obtenir le composant (A) de la composition d'agent d'amélioration de résistance pour une poudre hydraulique à partir du composant (a1) et du composant (a2) est un système réactionnel dans lequel un reflux de l'humidité est effectué et la température réactionnelle est de 80 °C ou moins.

7. Procédé de production d'une poudre hydraulique selon l'une quelconque des revendications 1 à 6, dans lequel au moins un composé sélectionné dans le groupe consistant en un adduit d'oxyde d'éthylène de glycérine, le diéthylène glycol, et la triéthanolamine est contenu lors de la pulvérisation du composé hydraulique.

8. Composition d'additif pour pulvériser un composé hydraulique, comprenant :
une composition d'agent d'amélioration de résistance pour une poudre hydraulique qui contient un produit réactionnel [ci-dessous dans le présent document, désigné par composant (A)] obtenu par la réaction (a1) de glycérine [ci-dessous dans le présent document, désignée par composant (al)] et (a2) de formaldéhyde [ci-dessous dans le présent document, désigné par composant (a2)] ; et
au moins un composé sélectionné dans le groupe consistant en un adduit d'oxyde d'éthylène de glycérine, le diéthylène glycol, et la triéthanolamine.

9. Utilisation d'une composition d'agent d'amélioration de résistance pour une poudre hydraulique comme adjuvant de pulvérisation lors de la pulvérisation d'un composé hydraulique, la composition d'agent d'amélioration de résistance comprenant un produit réactionnel [ci-dessous dans le présent document, désigné par composant (A)] obtenu par la réaction (a1) de glycérine [ci-dessous dans le présent document, désignée par composant (al)] et (a2) de formaldéhyde [ci-dessous dans le présent document, désigné par composant (a2)],
dans laquelle la composition d'agent d'amélioration de résistance est ajoutée à, en termes de matière solide, 0,0005 partie en masse ou plus et 1,0 partie en masse ou moins par rapport à 100 parties en masse du composé hydraulique.

10. Utilisation d'une composition d'agent d'amélioration de résistance pour une poudre hydraulique comme adjuvant de pulvérisation selon la revendication 9, dans laquelle la composition d'agent d'amélioration de résistance pour une poudre hydraulique comprend en outre au moins un composé sélectionné dans le groupe consistant en un condensat d'urée-formaldéhyde et un hydroxyméthane sulfonate [ci-dessous dans le présent document, désigné par composant (B)].

11. Utilisation d'une composition d'agent d'amélioration de résistance pour une poudre hydraulique comme adjuvant de pulvérisation selon la revendication 9 ou 10, dans laquelle le composé hydraulique comprend au moins une substance sélectionnée dans le groupe consistant en un laitier de haut fourneau, une cendre volante, et une fumée de silice à 10 % en masse ou plus et 80 % en masse ou moins.

12. Utilisation d'une composition d'agent d'amélioration de résistance pour une poudre hydraulique comme adjuvant de pulvérisation selon la revendication 10 ou 11, dans laquelle le rapport en masse entre le composant (B) et le composant (A), en termes de (B)/(A), est de plus de 0/100 et de 80/20 ou moins dans la composition d'agent d'amélioration de résistance pour une poudre hydraulique.

13. Utilisation d'une composition d'agent d'amélioration de résistance pour une poudre hydraulique comme adjuvant de pulvérisation selon l'une quelconque des revendications 9 à 12, dans laquelle un système réactionnel pour obtenir le composant (A) de la composition d'agent d'amélioration de résistance pour une poudre hydraulique à partir du composant (a1) et du composant (a2) est pour éliminer l'humidité du système à une température réactionnelle de 80 °C ou plus.

14. Utilisation d'une composition d'agent d'amélioration de résistance pour une poudre hydraulique comme adjuvant de pulvérisation selon l'une quelconque des revendications 9 à 12, dans laquelle un système réactionnel pour obtenir le composant (A) de la composition d'agent d'amélioration de résistance pour une poudre hydraulique à partir du composant (a1) et du composant (a2) est un système réactionnel dans lequel un reflux de l'humidité est effectué et la température réactionnelle est de 80 °C ou moins.

15. Utilisation d'une composition d'agent d'amélioration de résistance pour une poudre hydraulique comme adjuvant de pulvérisation selon l'une quelconque des revendications 9 à 14, dans laquelle la composition d'agent d'amélioration de résistance est utilisée comme adjuvant de pulvérisation pour pulvériser le composé hydraulique en présence d'au moins un composé sélectionné dans le groupe consistant en un adduit d'oxyde d'éthylène de glycérine, le diéthylène glycol, et la triéthanolamine.
